(19) Europäisches Patentamt / European Patent Office / Office européen des brevets

(11) **EP 3 722 444 B1**

(12) **EUROPEAN PATENT SPECIFICATION**

(45) Date of publication and mention
of the grant of the patent:
**05.06.2024 Bulletin 2024/23**

(21) Application number: **19169035.3**

(22) Date of filing: **12.04.2019**

(51) International Patent Classification (IPC):
**C12Q 1/6886** (2018.01)

(52) Cooperative Patent Classification (CPC):
**C12Q 1/6886;** C12Q 2600/118; C12Q 2600/158

(54) **METHOD FOR DETERMINING RCC SUBTYPES**

VERFAHREN ZUR BESTIMMUNG VON RCC-UNTERTYPEN

PROCÉDÉ DE DÉTERMINATION DE SOUS-TYPES RCC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.10.2020 Bulletin 2020/42**

(73) Proprietors:
• **Robert Bosch Gesellschaft für medizinische
Forschung mbH
70376 Stuttgart (DE)**
• **Eberhard Karls Universität Tübingen
Medizinische Fakultät
72074 Tübingen (DE)**
• **Friedrich-Alexander-Universität
Erlangen-Nürnberg
91054 Erlangen (DE)**

(72) Inventors:
• **BÜTTNER, Florian
70372 Stuttgart (DE)**
• **SCHÄFFELER, Elke
70376 Stuttgart (DE)**
• **SCHWAB, Matthias
70176 Stuttgart (DE)**
• **WINTER, Stefan
71522 Backnang (DE)**
• **BEDKE, Jens
72076 Tübingen (DE)**
• **STENZL, Arnulf
72076 Tübingen (DE)**
• **HARTMANN, Arndt
91054 Erlangen (DE)**

(74) Representative: **Witte, Weller & Partner
Patentanwälte mbB
Postfach 10 54 62
70047 Stuttgart (DE)**

(56) References cited:
WO-A2-2004/032842    CN-A- 108 410 988
US-A1- 2012 157 344    US-A1- 2013 157 887

• **TAKAHASHI MASAYUKI ET AL: "Molecular
subclassification of kidney tumors and the
discovery of new diagnostic markers",
ONCOGENE, NATURE PUBLISHING GROUP UK,
LONDON, vol. 22, no. 43, 2 October 2003
(2003-10-02), pages 6810-6818, XP002377970,
ISSN: 0950-9232, DOI: 10.1038/SJ.ONC.1206869**
• **"GeneChip Human Genome U133 Set",
INTERNET CITATION, 26 February 2003
(2003-02-26), XP002232760, Retrieved from the
Internet:
URL:http://www.affymetrix.com/support/tech
nical/datasheets/hgu133_datasheet.pdf
[retrieved on 1077]**
• **PETITPREZ FLORENT ET AL: "Transcriptomic
analysis of the tumor microenvironment to guide
prognosis and immunotherapies", CANCER
IMMUNOLOGY IMMUNOTHERAPY, SPRINGER,
BERLIN/HEIDELBERG, vol. 67, no. 6, 7 September
2017 (2017-09-07), pages 981-988, XP036502813,
ISSN: 0340-7004, DOI:
10.1007/S00262-017-2058-Z [retrieved on
2017-09-07]**

**Description**

The present invention relates to a method for determining in a subject's biological sample the relative proportions of papillary renal cell carcinoma (pRCC), clear cell renal cell carcinoma (ccRCC), and chromophobe renal cell carcinoma (chRCC), an array comprising capture molecules capable of specifically binding to RCC signature genes or coding sequences thereof or products encoded thereby, and to the use of RCC signature genes for classifying a subject into a renal cell carcinoma (RCC) risk group and/or for determining in a subject's biological sample the relative proportions of pRCC, ccRCC, and chRCC.

FIELD OF THE INVENTION

The present invention relates to a method for determining in a subject's biological sample the relative proportions of papillary renal cell carcinoma (pRCC), clear cell renal cell carcinoma (ccRCC), and chromophobe renal cell carcinoma (chRCC), an array comprising capture molecules capable of specifically binding to RCC signature genes or coding sequences thereof or products encoded thereby, and to the use of RCC signature genes for classifying a subject into a renal cell carcinoma (RCC) risk group and/or for determining in a subject's biological sample the relative proportions of pRCC, ccRCC, and chRCC.

BACKGROUND OF THE INVENTION

Renal cell carcinoma (RCC) comprises several histologically defined tumors that differ in biology, clinical course and response to treatment. The major subtypes are clear cell RCC (ccRCC), papillary RCC (pRCC), and chromophobe RCC (chRCC), which account for 65-70%, 15-20%, and 5-7% of all RCCs, respectively (Inamura, Trans-location Renal Cell Carcinoma: An Update on Clinicopathological and Molecular Features, Int. J. Mol. Sci. 9(9), p. 1-11 (2017)). In general, ccRCC has poor and chRCC has favorable prognosis. pRCC represents a heterogeneous group of RCC with intermediate prognosis compared to ccRCC and chRCC that has been subdivided in type 1 and type 2, a subset of tumors with mixed histology, and a small fraction of CpG island methylator phenotype (CIMP)-associated tumors (C.J. Ricketts et al., The Cancer Genome Atlas Comprehensive Molecular Characterization of Renal Cell Carcinoma, Cell Reports 23(1), p. 313-326 (2018)). Type 1 pRCC is associated with better prognosis than type 2 pRCC. CIMP tumors are characterized by poor survival.

Considering its significant prognostic as well as therapeutic implications, the correct determination of the subtype is of utmost importance. In clinical medicine, surgical specimen from RCC tumors are manually examined and classified by pathologists through histological and immunohistochemical analyses.

Pathological re-evaluation and bioinformatics analyses of molecular data have recently pointed to the shortcomings of pathological assessments of RCCs (Büttner et al., Survival Prediction of Clear Cell Renal Cell Carcinoma Based on Gene Expression Similarity to the Proximal Tubule of the Nephron, Eur. Urol. 68(6), p. 1016-1020 (2015); Chen et al., Multilevel Genomics-Based Taxonomy of Renal Cell Carcinoma, Cell Reports 14(10), p. 2476-2489 (2016); Schaeffeler et al., Metabolic and Lipidomic Reprogramming in Renal Cell Carcinoma Subtypes Reflects Regions of Tumor Origin, Eur. Urol. Focus (2018); C.J. Ricketts et al., *loc. cit.*). Manual classification is subjective and therefore bears potential for mislabeling or inconsistencies, especially in histologically ambiguous cases.

Rini et al., A 16-Gene Assay to Predict Recurrence After Surgery in Localised Renal Cell Carcinoma: Development and Validation Studies, Lancet Oncol. 16(6), p. 676-685 (2015), describe a prognostic multigene signature to improve prediction of recurrence risk in clear cell renal cell carcinoma. However, this method does not allow for an RCC subtype classification.

WO 2015/131095 discloses a method for distinguishing clear cell type A (ccA) renal cell carcinoma from clear cell type B (ccB) renal cell carcinoma in a subject. However, this method requires a statistically validated reference. Furthermore, it also does not allow for an RCC subtype classification beyond subtypes of ccRCC.

Wang et al., Identification and Validation of a 44-Gene Expression Signature for the Classification of Renal Cell Carcinomas, J. Exp. Clin. Cancer Res. 36:176, p. 1-11 (2017), disclose a 44-gene expression signature derived from microarray analysis which was associated with the histological differentiation of renal tumours and is proposed for tumor subtype classification. However, such gene expression signature has so far not proved successful in practice. Furthermore, the known method does not allow a subtype classification but only a clustering, i.e. individual biological samples cannot be classified.

Hence, there is a need for an objective subtype classification for RCC.

The present invention satisfies these and other needs.

SUMMARY OF THE INVENTION

The present invention provides a method for determining in a subject's biological sample the relative proportions of papillary renal cell carcinoma (pRCC), clear cell renal cell carcinoma (ccRCC), and chromophobe renal cell carcinoma (chRCC), the method comprising:

(a) Providing a biological sample from a subject suspected of being affected by RCC,

(b) Assaying said biological sample to determine expression level values of

- at least one of the signature genes listed in Table 1,

- at least one of the signature genes listed in Table 2, and

- at least one of the signature genes listed in Table 3,

(c) Subjecting the obtained expression level values to a signal separation method, thereby determining relative proportions of pRCC, ccRCC, and chRCC in said biological sample.

The inventors have developed an objective RCC subtype classification system based on gene expression data by which the disadvantages of the methods known in the art can be reduced or even avoided. The present invention can also be used to separate tumors that can be unambiguously assigned to the three major histological subtypes from those combining features from different subtypes. The method according to the invention also allows a new classification of RCC risk groups that is significantly stronger associated to survival outcome than common pathological classification.

The present invention is superior to currently performed manual histopathological classification because (1) it provides a precise and objective molecular-based procedure to classify RCC, (2) it quantifies the proportions of the major subtypes in histologically ambiguous RCC, and (3) the predicted proportional subtype composition is directly associated to a prognostic estimate.

The term "subject" as used herein refers to a member of any invertebrate or vertebrate species. Accordingly, the term "subject" is intended to encompass any member of the Kingdom Animalia including, but not limited to the phylum *Chordata* (i.e., members of classes *Osteichythyes* (bony fish), *Amphibia* (amphibians), *Reptilia* (reptiles), *Aves* (birds), and *Mammalia* (mammals)), and all orders and families encompassed therein. In an embodiment, the subject is a human.

A "biological sample" as used herein refers to biological material originating from the subject and comprises nucleic acids, and/or proteins, and/or peptides and /or polypeptides and/or fragments thereof. In an embodiment of the invention the biological sample comprises cellular material, cells or tissues. Preferably, the biological material comprises cells suspected of including renal carcinoma cell(s) or cells being renal carcinoma cell(s). In the clinical routine the biological sample may be a biopsy sample taken from potentially tumorous or RCC tissue, blood plasma, urine etc.

The terms "nucleic acid molecule" and "nucleic acid" refer to deoxyribonucleotides, ribonucleotides, and polymers thereof, in single-stranded or double-stranded form. As used herein, the terms "peptide" and "polypeptide" refer to polymers of at least two amino acids linked by peptide bonds. Typically, "peptides" are shorter than "polypeptides" and the latter are typically shorter than proteins, but unless the context specifically requires, these terms are used interchangeably herein.

As used herein the term "gene" refers to a hereditary unit including a sequence of DNA that occupies a specific location on a chromosome and that contains the genetic instruction for a particular characteristic or trait in an organism. Similarly, the phrase "gene product" refers to biological molecules that are the transcription and/or translation products of genes. Exemplary gene products include, but are not limited to mRNAs and polypeptides that result from translation of mRNAs.

A "signature gene" as used herein refers to a gene listed in any of Table 1, 2, and 3 and being indicative for pRCC (Table 1), ccRCC (Table 2), and chRCC (Table 3), respectively. The signature genes as referred to herein make up a so-called gene signature with a unique pattern of gene expression which is characteristic in cells of ccRCC, pRCC, and chRCC.

The signature genes can be clearly identified in Tables 1, 2, and 3 by means of their GeneID, i.e. the first column of the respective table. GeneID is a unique identifier that is assigned to a gene record in the meta search engine or database 'Entrez Gene' operated by the National Center for Biotechnology Information (NCBI). Synonyms for 'GeneID' are Gene Identifies (NCBI), NCBI gene ID, Entrez gene ID, NCBI geneid, or Gene identifier (Entrez). The 'Symbol' column lists the HUGO Gene symbols of the genes. The columns headlined 'ccRCC', 'chRCC', and 'pRCC' list relative expression values of the respective signature genes in the indicated RCC subtypes. The expression values are (unlogtransformed) processed signal intensities measured with the Affymetrix HTA2.0 array.

As used herein "at least one" signature gene refers to the minimum of one signature gene of each Table or group that needs to be analyzed. In embodiments of the invention 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, or all 102 signature genes of each Table or group are analyzed in respect of their expression levels. Further, in embodiments of the invention the numbers of signatures genes can be the same or different from each of the Tables or groups, i.e. x genes out of Table 1, y genes out of Table 2, and z genes out of Table 3 can be analyzed, while $x$, $y$, and z stand for the same or different integers.

While, according to the findings of the inventors, the analysis of one signature gene per Table is sufficient for a determination of the relative proportions of pRCC, ccRCC, and chRCC in the biological sample, the accuracy of determination and the reliability of the method is increasingly enhanced by the inclusion of more than one signature gene up to all 102 signature genes of each Table or group, respectively.

As is known to one of ordinary skill in the art, gene expression levels can be assayed at the level of RNA and/or at the level of protein. As such, in some embodiments RNA is extracted from the biological sample and analyzed by techniques that include, but are not limited to, PCR analysis (in some embodiments, quantitative reverse transcription PCR), nucleotide sequencing and/or array analysis. Alternatively or in addition, gene expression levels can be assayed by determining the levels at which proteins or polypeptides are present in the biological sample. This can also be done using arrays, and exemplary methods for producing peptide and/or polypeptide arrays attached to a suitable carrier are well known to the skilled person. In each case, one of ordinary skill in the art would be aware of techniques that can be employed to determine the expression level of a gene in the biological sample.

A "signal separation method" as used herein refers to a process for the analysis of mixtures of signals with the objective to recover the original component signals from the mixture. In particular, it is referred to a method for determining the relative proportions of ccRCC, pRCC, and chRCC in said biological sample. It includes methods like blind signal separation (BSS) such as deconvolution, principal component analysis (PCA), independent component analysis (ICA), machine learning (supervised learning/classification/regression) and data mining (unsupervised learning/clustering).

The object underlying the invention is herewith completely solved.

The inventors have realized that determining the expression level values of only at least one of the signature genes listed in each of Tables 1, 2, and 3, i.e. of only at least three different genes, and subjecting the obtained expression level values to a signal separation method allows the determination of relative proportions of pRCC, ccRCC, and chRCC in the biological sample.

The method according to the invention allows an objective determination of the RCC subtype, thereby avoiding an incorrect subjective classification made by a pathologist. Another advantage of the method according to the invention over the methods in the art is that no reference is required to allow a correct subtype classification.

Knowledge of a pRCC versus ccRCC and chRCC class assignment allows for an assessment of risk for recurrence or cancer specific death, and can be used to augment clinical information to make more accurate risk assessments. Knowledge of risk, allows clinicians to tailor the post-operative evaluations, and to consider adjuvant therapy options. Particular changes to care that might arise when a subject's RCC is classified as comprising significant proportions of any of pRCC, ccRCC and chRCC might include, but not be limited to more intensive monitoring, consideration of surgical intervention, drug/radiation therapy, and/or finding an adjuvant therapy trial for the subject to reduce risk for recurrence.

In an embodiment of the method according to the invention in step (b) said biological sample is assayed to determine expression level values of at least two of the signature genes listed in Table 1, at least two of the signature genes listed in Table 2, and at least two of the signature genes listed in Table 3.

This measure has the advantage that the accuracy of the determination of the relative proportions of pRCC, ccRCC, and chRCC in said biological sample is further increased.

In another embodiment of the present invention the signal separation method is a blind signal separation method (BSS).

Blind signal separation (BSS), also known as blind source separation, refers to a method for the separation of a set of source signals from a set of mixed signals, without the aid of information (or with very little information) about the source signals or the mixing process. The inventors have realized that BSS, if used in the method of the invention, allows a high degree of signal separation and ensures the achievement of reliable results.

In a further embodiment of the method according to the invention the blind separation method is deconvolution, preferably computational deconvolution.

Deconvolution is an algorithm-based process used to reverse the effects of convolution on recorded data. Initially, deconvolution has been mainly used in the techniques of signal processing and image processing. Computational deconvolution refers to a computer-assisted decovolution method which has been used to address specific questions of biology or bioinformatics, as e.g. described in S. S. Shen-Orr and R. Gaujoux, Computational Deconvolution: Extracting Cell Type-Specific Information from Heterogeneous Samples, Current Opinion in Immunology 25, p. 571-578 (2013);

F. Avlia Cobos et al., Computational Deconvolution of Transcriptomics Data from Mixed Cell Populations, Bioinformatics 34, p. 1969-1979 (2018); A. R. Abbas et al., Deconvolution of Blood Microarray Data Identifies Cellular Activation Patterns in Systemic Lupus Erythema-tosus., PloS one 4, e6098 (2009); R. Gaujoux and C. Seoighe, CellMix: A Comprehensive Toolbox for Gene ExpressionDeconvolution., Bioinformatics (Oxford, England), p. 1-2 (2013). The inventors, however, realized for the very first time that the deconvolution method can be used in an advantageous manner to determine in a heterogeneous biological or RCC sample the relative proportions of the respective RCC subtypes.

In an embodiment of the present invention after step (c) the following step is carried out: Classifying the subject into a risk group on the basis of the relative proportions of ccRCC, pRCC, and chRCC in said biological sample.

The inventors have realized that by the developed inventive concept not only the relative proportions of the respective RCC subtypes can be determined in a biological or RCC sample but also a prognosis in terms of allocating the patient to a risk group can be made. This measure implements the invention into the clinic in an advantageous manner.

In another embodiment of the present invention the risk group is selected from "good", "intermediate", and "poor" according to the prognosis for the subject.

This measure allows a rapid allocation of a prognosis for the affected subject in daily hospital routines. "Good" refers to a high likelihood of the subject to survive for more than 5 years, "poor" refers to a low likelihood of the subject to survive for more than 5 years, and "intermediate" refers to a medium likelihood of the subject to survive for more than 5 years, each 5 years period beginning to run at the date of the initial diagnosis. In an embodiment of the invention in the "good" group the likelihood is about 85-95% or higher, preferably 92%, in the "poor" group the likelihood is about 45-55%, preferably 49%, and in the "intermediate" group the likelihood is about 70-80%, preferably 77%.

In another embodiment of the invention the "good" group is determined by a relative ccRCC proportion of about $\leq 50\%$, further preferably about $\leq 45\%$, further preferably about 40%, further preferably about $\leq 35\%$, further preferably about $\leq 30\%$, further preferably about $\leq 25\%$, further preferably about $\leq 20\%$, further preferably about $\leq 15\%$, and highly preferably about < 13.7%.

In yet another embodiment of the invention the "intermediate" group is determined by a relative pRCC proportion of about $\leq 50\%$, further preferably about $\leq 45\%$, further preferably about $\leq 40\%$, further preferably about $\leq 35\%$, further preferably about $\leq 30\%$, further preferably about $\leq 25\%$, further preferably about $\leq 20\%$, and highly preferably of about $\leq 16.5\%$.

In still another embodiment of the invention the "poor" group is determined by a relative pRCC proportion of about $\geq 5\%$, preferably about $\geq 7.5\%$, further preferably about $\geq 10\%$, further preferably about $\geq 12\%$, and highly preferably about > 16.5%.

The inventors have realized that the indicated thresholds of the relative proportions of the respective RCC subtypes allow an allocation of the subject to a risk group "good", "poor", and/or "intermediate". It is accepted that by using the rough or less-specific thresholds each mentioned for the less preferred embodiments a subject may fall into more than one risk group. However, it is clear that the more specific thresholds each mentioned for the more or further preferred embodiments allow an increasingly distinctive allocation of a subject to a specific risk group.

In another embodiment of the invention in step (b) the assaying involves the use of RNA sequencing, a PCR-based method, a microarray-based method, a hybridization-based method and/or an antibody-based method.

This measure takes advantage of such methods for assaying the biological sample which have been proven their suitability for determining expression level values of genes or gene products.

The methods can be carried out with an array comprising capture molecules capable of specifically binding to

- biomolecules encoding or encoded by at least one, preferably at least two of the signature genes listed in Table 1 or segments thereof,

- biomolecules encoding or encoded by at least one, preferably at least two of the signature genes listed in Table 2 or segments thereof, and

- biomolecules encoding or encoded by at least one, preferably at least two of the signature genes listed in Table 3.

The "biomolecules" include, but are not limited hereto, nucleic acid molecules encoding the signature genes, proteins, peptides or polypeptides encoded by the signature genes. The "capture molecules" include, but are not limited hereto, nucleic acid molecules (e.g. hybridization probes, aptamers etc.), antibodies and fragments thereof.

The term "array" is to be understood in its broadest sense and refers to any kind of test format suitably adapted to comprise the capture molecules and to carry out a binding reaction of the signature genes or gene products or equivalents to the capture molecules. Preferably, the array is a microarray.

Another subject-matter of the present invention is the use of

- at least one, preferably at least two of the signature genes listed in Table 1,

- at least one, preferably at least two of the signature gene listed in Table 2, and
- at least one, preferably at least two of the signature genes listed in Table 3,

for classifying a subject into a renal cell carcinoma (RCC) risk group.

Another subject- matter of the present invention is the use of

- at least one, preferably at least two of the signature genes listed in Table 1,

- at least one, preferably at least two of the signature genes listed in Table 2, and

- at least one, preferably at least two of the signature genes listed in Table 3,

for determining in a subject's biological sample the relative proportions of papillary renal cell carcinoma (pRCC), clear cell renal cell carcinoma (ccRCC), and chromophobe renal cell carcinoma (chRCC), further preferably for classifying the subject into a RCC risk group.

The embodiments, features, characteristics, and advantages disclosed for the method according to the invention apply likewise to the uses according to the invention.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention.

Certain features described in the context of various embodiments are not to be considered essential features of those embodiments, unless the embodiment is inoperative without those elements.

It is to be understood that the before-mentioned features and those to be mentioned in the following cannot only be used in the combination indicated in the respective case, but also in other combinations or in an isolated manner without departing from the scope of the invention.

The invention is now described and explained in further detail by referring to the following non-limiting examples and drawings.

BRIEF DESCRIPTION OF THE DRAWINGS

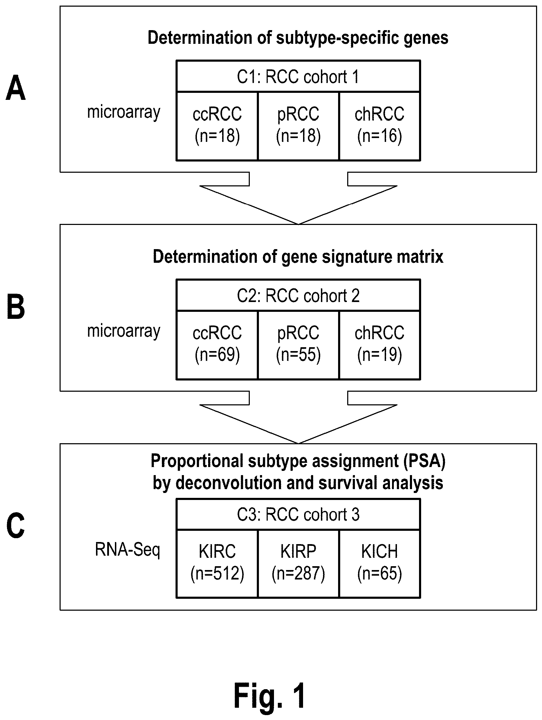

Figure 1: Overview about the workflow of data analysis including different cohorts and RNA quantification technologies (microarray and RNA-Seq) used in the development of the present invention.

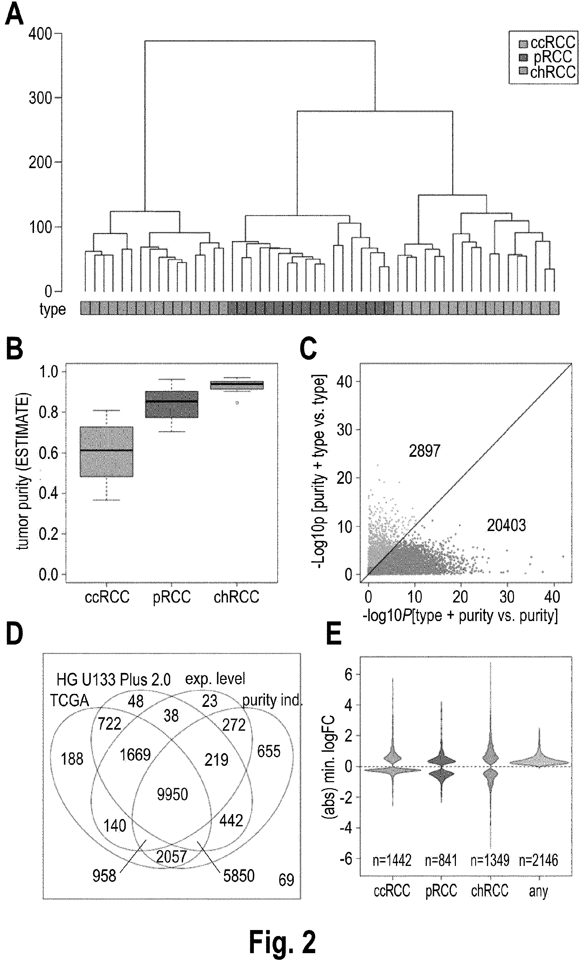

Figure 2: Selection of candidate genes for the signature matrix using cohort C1. (A) Hierarchical clustering of cohort C1 (n=52) using Ward's method. (B) Tumor purity as determined by the ESTIMATE method varies between pathological subtypes. (C) The scatter plot shows P-values obtained from model comparison for each gene. It was tested if expression is better explained by tumor purity or subtype. 20403 genes were stronger associated to tumor purity. (D) 9950 genes remained after four filtering steps. (E) Log fold changes of subtype-specific genes obtained by analysis of variance and subsequent post-hoc testing using Tukey's method. For each gene and subtype the minimum log fold change compared to the two respective other subtypes was considered here. "Any" includes genes that were differently expressed between any pair of subtypes. These genes were not further used in this work.

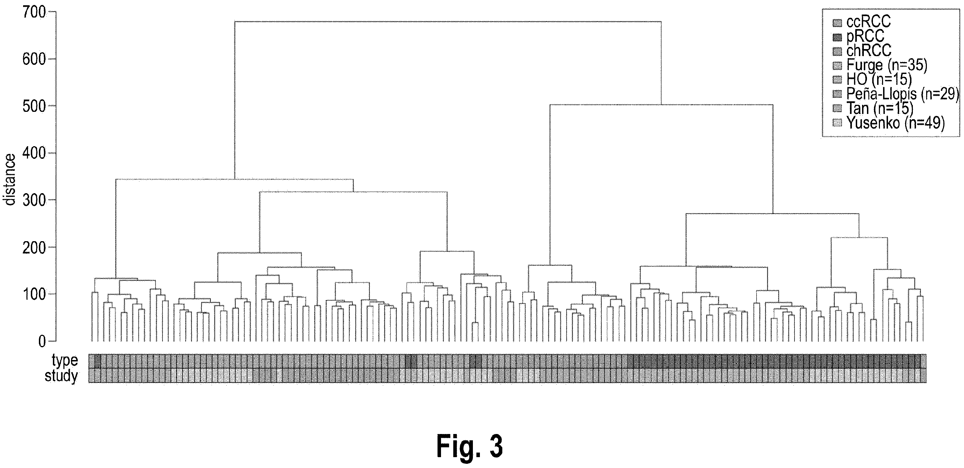

Figure 3: Hierarchical clustering of cohort C2. Hierarchical clustering of cohort C2 (n=143) using Ward's method. Cohort C2 was a combined cohort containing RCC samples from five different studies (Table S1).

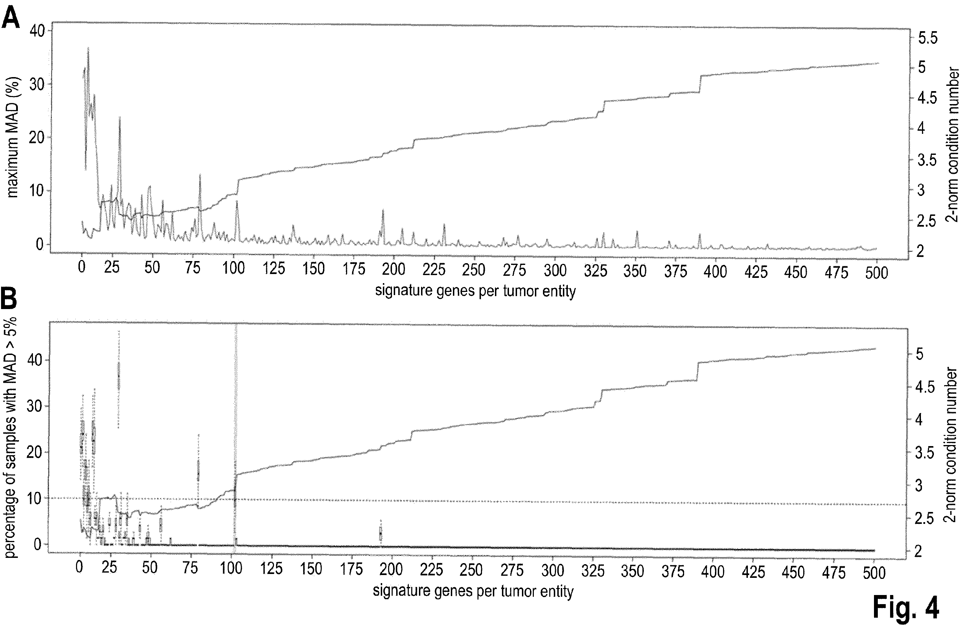

Figure 4: Signature matrices with increasing number of genes were tested. The initial matrix included the top five genes per subtype exhibiting the highest log fold change compared to the respective other subtypes (Fig. 2E). The median gene expression per subtype based on cohort C1 was used. Matrix sizes ranged from 15, i.e. the top five genes per subtype, to 1500 genes. Each matrix was used to deconvolve the 143 transcriptomes from cohort C2. The maximum absolute difference (MAD) was computed between consecutive matrices for each sample. The blue line depicts the 2-norm condition number of the matrices. (For details see Material & Methods.) (A) The maximum MAD between two consecutive matrices is shown. (B) 50% subsets were randomly drawn 10000 times from cohort C2 and for each tested matrix and sample subset the percentage of samples experiencing a MAD > 5% compared to the predecessor matrix was determined. Matrix *RCC102* including the top 102 genes per subtype was chosen (marked in light red).

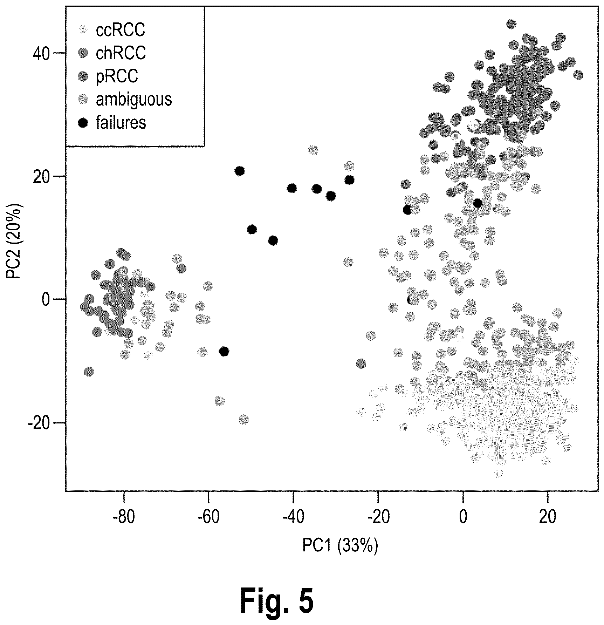

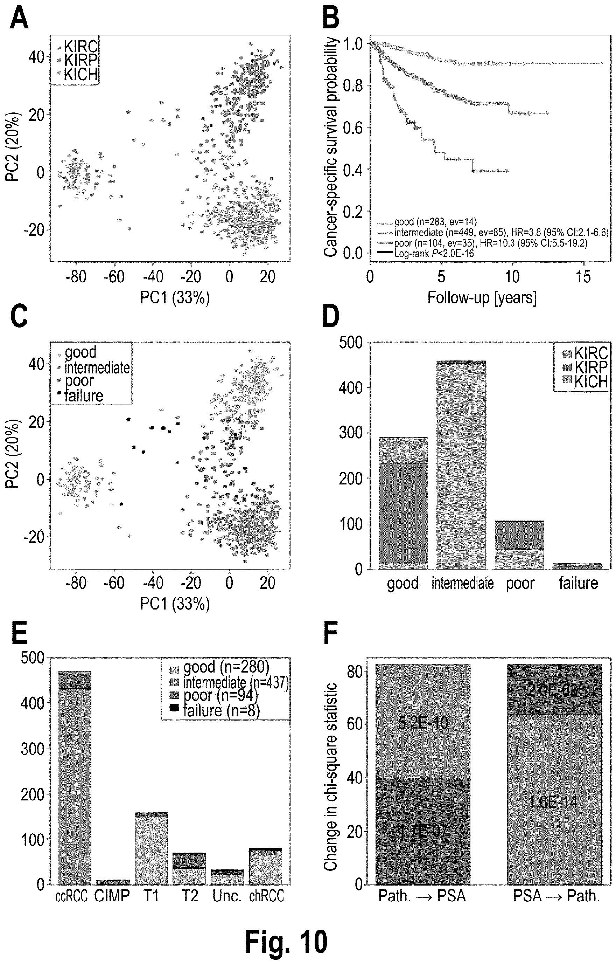

Figure 5: Clear and ambiguous cases in TCGA RCC cohort (C3). Samples with maximum assigned proportion < 95% by deconvolution were considered ambiguous here and are colored in grey, compare with Figure 10A. 11 samples for which *RCC 102 was* not valid to estimate their PSA are colored in black.

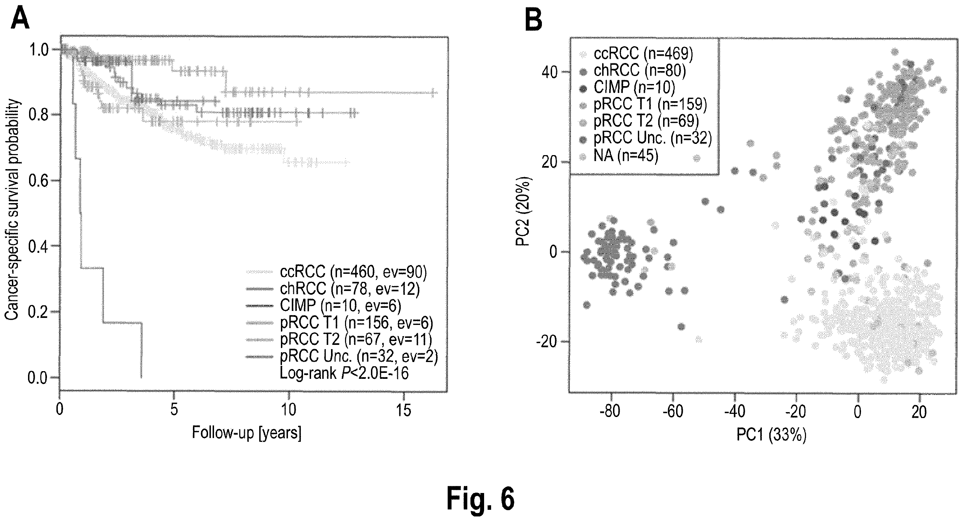

Figure 6: Pathological classification of TCGA RCC cohort (C3). (A) Kaplan-Meier curves showing cancer-specific survival (CSS) of pathological subtypes (C.J. Ricketts et al., *loc.cit.*) in cohort C3 (n=803). (B) PCA plot from Figure 2A with samples colored according to their pathological subtype. 45 tumors from C3 were not included in (C.J. Ricketts et al., *loc. cit.*).

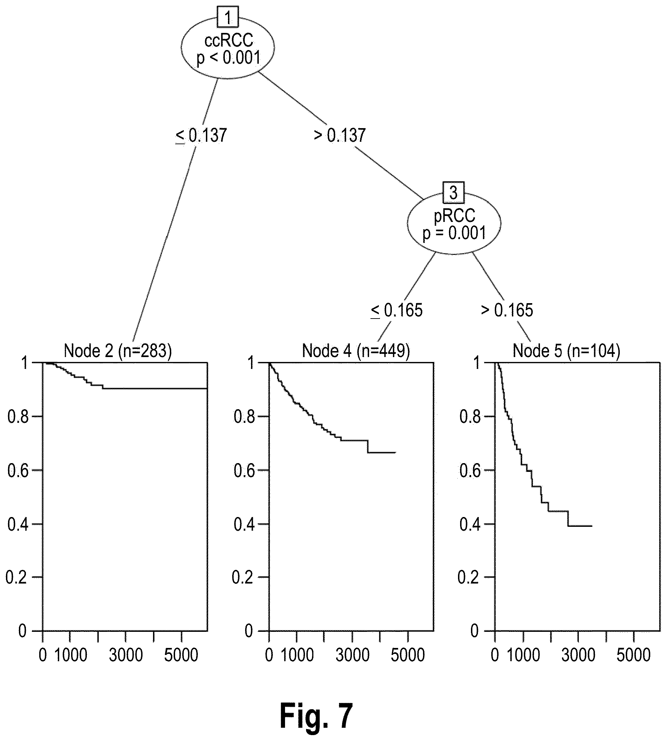

Figure 7: Definition of RCC risk groups based on proportional subtype assignments (PSA) using conditional inference trees. Conditional inference trees were used to search for groups in the TCGA RCC cohort (C3) with significantly varying survival probability based on PSA. The method identified 3 groups termed "good", "intermediate" and "poor" (from left to right). Samples with less than 13.7% ccRCC proportion are sorted into the "good" group. Remaining samples are classified based on their pRCC proportion.

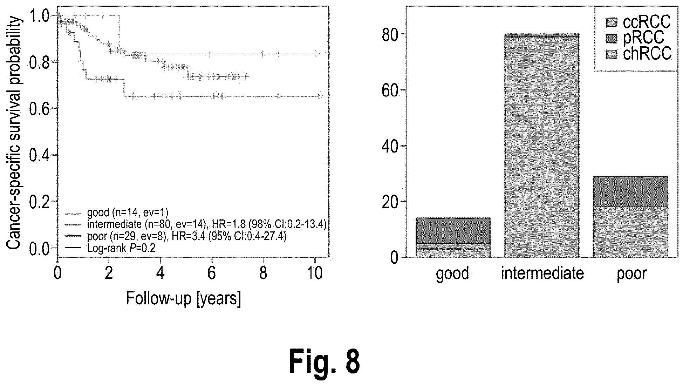

Figure 8: Association to CSS of proportional subtype assignments (PSA) using different signature gene subsets.

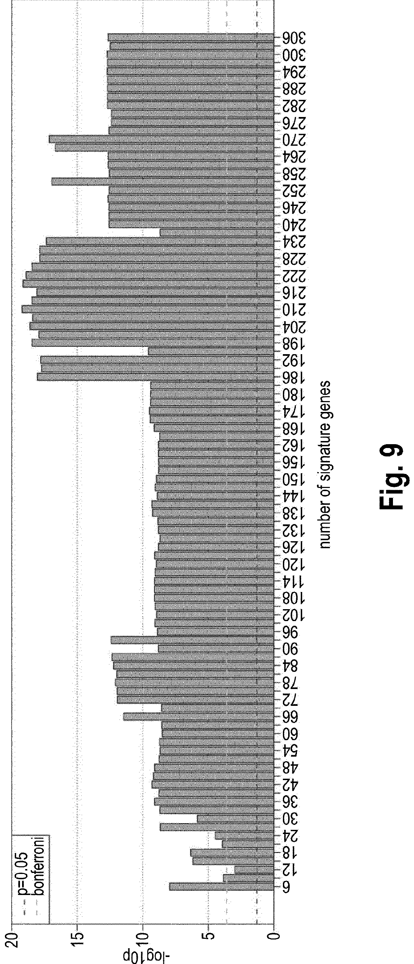

Figure 9: (A) Principal component analysis of TCGA RCC cohorts (cohort C3). Using the 306 genes included in the established RCC signature matrix *(RCC102),* a principal component analysis was carried out with the 864 samples from the combined TCGA RCC cohort, including cohorts KIRC, KIRP, and KICH. (B) Cancer-specific survival (CSS) of PSA-based risk groups. Kaplan-Meier curves showing CSS of cohort C3 (n=836). 28 samples were disregarded due to lack of survival data or non-validity of the deconvolution approach. Risk groups were determined based on PSA by conditional inference tree models for endpoint CSS. (C, D) Comparison between PSA-based risk groups and original TCGA classification. (C) PCA plot from (A) with samples colored according to their risk group membership. 11 samples for which *RCC102 was* not valid to estimate their PSA are colored black (failure). (D) Bar plot showing how TCGA samples from KIRC, KIRP and KICH are distributed on PSA-based risk groups. (E) Comparison between PSA-based risk groups and updated TCGA classification (C.J. Ricketts et al., *loc. cit.*). Bar plot showing how risk groups are distributed on updated pathological classification of C3. 819 samples from C3 were included in (C.J. Ricketts et al., *loc. cit.*). T1=pRCC T1, T2=pRCC T2, Unc.=pRCC Unc. (F) Prognostic prediction based on PSA significantly improves pathological classification. Chi-square statistic values depict the improvement of the model likelihood when risk classification based on PSA (red) was added to the Cox model initially including only the pathological classification (blue; left) or vice versa (right). Chi-squared test P-values are shown in the bars. 795 samples with available survival data from (E) were involved in this model comparison.

EXAMPLES

1. Overview

A subtype classification system based on gene expression data was developed for renal cell carcinoma (RCC). The basic idea was to model any RCC sample as a linear combination of clear cell RCC (ccRCC), chromophobe RCC (chRCC) and papillary RCC (pRCC). More than 95% of all RCC are assigned to one of these subtypes based on histological analysis and they represent both proximal and distal cell types as origin of kidney cancer evolution. Essentially, the inventors assumed a tumor not necessarily belonging to only one of these subtypes, but to carry parts of each of them. Therefore, rather than categorizing a tumor, the inventors intended to break down its composition through proportional subtype assignments (PSA).

The inventors realized that signal separation, in particular computational deconvolution represented the method of choice for this problem. Gene expression deconvolution has been successfully applied to characterize the cell composition of heterogeneous samples, e.g. peripheral blood that includes many different immune cell types (S.S. Shen-Orr and R. Gaujoux, *loc. cit.*). Here, RCC were modeled as heterogeneous tissues that are composed of varying proportions of ccRCC, chRCC and pRCC.

This study did groundwork by attempting for the first time to comprehensively detect and quantify clear as well as composed signals in RCC samples indicative of the tumor type. Methodologically, an unsupervised approach has been developed to utilize yet unknown patterns in gene expression profiles of RCC samples for subtype classification.

The inventors' approach is able to separate RCC tumors that can be unambiguously assigned to one of the main histological subtypes from those evading a clear histological classification. Unclear tumors were described as mixed types that combine features from different subtypes. Further, PSA enabled a new definition of RCC risk groups that is significantly stronger associated to survival outcome than common pathological classification. Concluding, PSA as determined by the method according to the invention simplifies classification of RCC and specifies prognosis.

2. Material and Methods

Patient cohorts

Figure 1 shows to what purpose the three cohorts were used in each case.

RCC cohort 1 (C1) consisted of 52 primary tumor samples with either clear cell (n=18), papillary (n=18), or chromophobe RCC histology (n=16). All of these were collected from patients treated at the Department of Urology, University Hospital Tuebingen, Germany. Use of the tissue was approved by the ethics committee of the University of Tuebingen and informed written consent was provided by each subject prior to surgical resection. Surgically resected ccRCC tissues were classified according to the seventh edition of the Union Internationale Contre le Cancer/American Joint Committee on Cancer system (2009). None of the patients received any kind of neoadjuvant therapy before surgery, neither immune- nor chemotherapy. Importantly, these samples have been independently evaluated by two teams of pathologists with special expertise in nephro-pathology to have maximum certainty regarding their RCC subtype. C1 was used to identify genes with RCC subtype-specific expression (Fig. 2A).

RCC cohort 2 (C2) is a combined cohort containing 143 RCC samples from five studies (K.A. Furge et al., Detection of DNA Copy Number Changes and Oncogenic Signaling Abnormalities from Gene Expression Data Reveals MYC Activation in High-Grade Papillary Renal Cell Carcinoma. Cancer Res. 67(7), p. 3171-3176 (2007); M.-H. Tan et al., Genomic Expression and Single-Nucleotide Polymorphism Profiling Discriminates Chromophobe Renal Cell Carcinoma and Oncocytoma. BMC Cancer, 10:196 (2010); S. Peña-Llopis et al., BAP1 Loss Defines a New Class of Renal Cell Carcinoma. Nat. Genet. 44(7), p. 751-759 (2012); M.V. Yusenko et al., High-resolution DNA Copy Number and Gene Expression Analyses Distinguish Chromophobe Renal Cell Carcinomas and Renal Oncocytomas. BMC Cancer 9, p. 152 (2009); T.H. Ho et al., Differential Gene Expression Profiling of Matched Primary Renal Cell Carcinoma and Metastases Reveals Upregulation of Extracellular Matrix Genes. Ann. Oncol. Off. J. Eur. Soc. Med. Oncol. 28(3), p. 604-10 (2017)). Common to these studies was the use of the Affymetrix GeneChip HG U133 Plus 2.0 for quantification of gene expression. Only samples from primary tumor tissue that were labeled ccRCC, chRCC or pRCC in the original study were added to C2. Table S1 shows the numbers of samples per subtype obtained from each study. C2 was used to determine the signature (Fig. 3).

**[0071]** **Table S1:** Cohort C2 (n=143) includes RCC samples from five studies providing gene expression data on Gene expression omnibus. Expression measurements were conducted with Affymetrix microarray HG U133 Plus 2.0.

| Study | GEO accession | ccRCC | pRCC | chRCC |
|---|---|---|---|---|
| Yusenko et al. | GSE11151 | 26 | 19 | 4 |
| Tan et al. | GSE19982 | 0 | 0 | 15 |
| Peña-Llopis et al. | GSE36895 | 29 | 0 | 0 |
| Furge et al. | GSE7023 | 0 | 35 | 0 |
| Ho et al. | GSE85258 | 14 | 1 | 0 |

Using the established signature matrix, samples from the TCGA RCC cohort (C3) were deconvolved. Clinical information and gene expression data ("FPKM-UQ") generated by RNA-Seq from kidney cancer cohorts KIRC, KICH and KIRP from TCGA were downloaded on November 30, 2017 from https://gdc.cancer.gov/ using R-package TCGAbiolinks. XML-structured clinical information was processed using R-package XML. Disease-specific survival outcome data for the TCGA RCC cohort was obtained from (J. Liu et al., An Integrated TCGA Pan-Cancer Clinical Data Resource to Drive High-Quality Survival Outcome Analytics. Cell 173(2):400-416.e11 (2018) and was referred to as CSS in this work. 17 KIRC patients that received prior treatment for their disease were excluded. Four patients of the KIRC cohort were represented by several samples in the expression data set. The sample with highest median expression was chosen, respectively. To be sure that only tumor samples were included, remaining tumor and non-tumor samples from the TCGA RCC cohort were hierarchically clustered using Ward's method. 2 cases (TCGA-BQ-5899, TCGA-CJ-5683) that were wrongly assigned to be tumor were excluded. In case of TCGA-CW-5591 tumor and non-tumor data had been confused. In total the TCGA RCC cohort included tumor samples from 864 patients (KIRC: 512, KIRP: 287, KICH: 65) and for 847 of which cancer-specific survival (CSS) information was available.

Preprocessing of gene expression data

High quality total RNA was isolated from fresh-frozen RCC tissue from cohorts C1 and C4 using the mirVana™ miRNA Isolation Kit (Life Technologies) as previously described (P. Fisel et al., DNA Methylation of the SLC16A3 Promoter Regulates Expression of the Human Lactate Transporter MCT4 in Renal Cancer with Consequences for Clinical Outcome. Clin. Cancer Res. 19(18), p. 5170-5181 (2013), S. Winter et al., Methylomes of Renal Cell Lines and Tumors or Metastases Differ Significantly with Impact on Pharmacogenes. Sci Rep. 6(1) (2018). Genome-wide transcriptome analyses were performed using the Human Transcriptome Array HTA 2.0 (Affymetrix) according to the manufacturer's protocol. Further processing of microarray data were performed as previously described (S. Winter et al., *loc. cit.*). Array quality control was conducted by Affymetrix Expression Console (Build 1.4.1.46). The microarrays from C1 and C4, respectively, were preprocessed together using the Robust Multiarray Average (RMA) implementation from the R-package oligo.

Genome-wide transcriptome measurements performed by Affymetrix GeneChip HG U133 Plus 2.0 from 143 RCC patients included in C2 were downloaded from Gene Expression Omnibus (GEO) using R-package GEOquery (Table S1). Microarrays from C2 were preprocessed together using the RMA implementation from the R-package oligo.

Entrez GeneIDs were used as gene identifiers in this work. Probeset measurements corresponding to a single gene were collapsed by taking the median expression. For HTA 2.0 measurements probeset annotations included in Bioconductor package hta20transcriptcluster.db were used. Annotation data from GEO (GPL570) were applied for summarizing expression data generated with Affymetrix GeneChip HG U133 Plus 2.0. Ensembl gene identifiers used in TCGA expression data were mapped to Entrez GeneIDs by means of the org.Hs.eg.db annotation package.

Statistical tools

All statistical analyses were performed with R-3.4.4 including additional packages MASS_7.3-49, partykit_1.2-0, squash_1.0.8, survival_2.41-3, and XML_3.98-1.10., GEOquery_2.46.15, hta20transcriptcluster.db_8.7.0, oligo_1.42.0, org.Hs.eg.db_3.5.0, SummarizedExperiment_1.8.1, and TCGAbiolinks_2.6.12 are part of the Bioconductor software project (http://www.bioconductor.org). All statistical tests were two-sided. Statistical significance was defined as P-value < 0.05.

In hierarchical cluster analyses Euclidean distance and Ward's method have been used if not stated otherwise.

Survival analyses

Cancer-specific survival (CSS) was used as an endpoint in survival analyses involving cohort C3. CSS was defined as the time from initial diagnosis to death or last date of follow-up if alive. Data for patients who died from other causes than RCC disease were considered censored at the time of death. Survival analyses were conducted by Kaplan-Meier curves and corresponding log-rank tests. Comparisons of Cox models were performed by analysis of deviance. Using the conditional inference tree framework with endpoint CSS from the R-package partykit cohort C3 was partitioned based on subtype assignments (Figure 7).

Gene expression deconvolution using robust linear regression

In this work, RCC samples were considered as mixtures of ccRCC, chRCC and pRCC. Further, it was assumed that the proportional composition of the three main subtypes is reflected in the gene expression profile of the mixed samples. According to the linearity assumption (Y. Zhao and R. Simon, Gene Expression Deconvolution in Clinical Samples. Genome Med. 2(12), p. 93 (2010)), the expression of each gene in a mixed RCC sample can thus be modeled as weighted average of the expression of this gene in ccRCC, chRCC and pRCC. The weights correspond to the respective proportional composition that can be estimated by gene expression deconvolution.

The objective of deconvolution is to find a solution to the system of linear equations: $m = s \cdot f$. Here, vector $f$ denotes the unknown proportions of ccRCC $c$, chRCC $h$ and pRCC $p$ in a mixed sample A. $m$ represents the mRNA mixture of A. $s$ is a gene signature matrix including the expression values of the signature genes in the three RCC subtypes. Signature genes were defined based on a set of ccRCC, chRCC and pRCC samples that could be uniquely assigned by pathologists or previous analyses of molecular data. The matrix equation can be solved for $f$ using standard linear least squares regression (A.R. Abbas et al., *loc. cit.*). However, to increase stability of subtype assignments robust linear regression as implemented in the "rlm" function from the R-package MASS (parameter maxit was set to 200) was used in this work. Expression deconvolution was performed on linear, i.e. non-log-transformed, expression data as suggested by (Y. Zhong and Z. Liu, Gene expression deconvolution in linear space. Nat. Methods 9(1), p. 8-9 (2011). Further, linear expression values were centered to zero mean and scaled to unit variance preceding deconvolution. Negative regression coefficients were set to 0 and percentages were calculated by dividing the three estimates by their sum, such that $c + p + h$ = 100%.

An empirical P-value was calculated to test the ability of the signature to discover RCC subtypes in a certain RCC sample. Basically, the P-value estimation was carried out in the same way as described in (A.M. Newman et al., Robust enumeration of cell subsets from tissue expression profiles. Nat. Methods 12(5), p. 453-457 (2015).). Briefly, the Pearson correlation $R$ between $m$ and $s \cdot f$ was compared against a derived null distribution $R^*$ for sample A. Expression values in $m$ were replaced by randomly drawn values from the full transcriptome of A, denoted $m_i^*$. Subtype proportions $f_i^*$ were determined for $m_i^*$ by deconvolution and the Pearson correlation between $m_i^*$ and $s \cdot f_i^*$ was calculated. This process was repeated 999 times, yielding $R^*$, and the P-value was obtained by $(|R^* > R| + 1)/(999 + 1)$.

## Selection of candidate genes

Samples from C1 were considered as clear cases since they could unambiguously be assigned to one of the main subtypes (Fig. 2A). Transcriptome-wide RNA expression in C1 was quantified using Human Transcriptome Array 2.0 microarray technology comprising 23300 genes.

First, genes with consistently low expression were removed. To have all samples at the same expression level they were median centered before and only for this filter step. Only genes that showed in at least one of the three tumor entities a median expression above the global median, i.e. zero, were kept. Further, genes not covered in TCGA RNA expression data or the Human Genome U133 Plus 2.0 Array were excluded. It has been demonstrated by (K. Yoshihara et al., Inferring tumour purity and stromal and immune cell admixture from expression data. Nat. Commun. 4, p. 2612 (2013)) using TCGA data that RCC subtypes vary in tumor purity, see http://bioinformatics.mdanderson.org/estimate/. This pattern could be observed in the cohort C1 as well (Fig. 2B). To minimize dependence on tumor purity genes stronger related to tumor purity, as determined by the ESTIMATE method, than to tumor type were removed. To be precise, for each gene linear regression models were fit incorporating either tumor type or tumor purity as single predictor or both of them in a multiple regression model. In the latter case models with and without interaction effects were considered. The reduction in the residual sum of squares was compared by analysis of deviance tests. Per feature, i.e. purity or type, main and main + interaction effect was tested and the lower P-value was selected. A gene was kept in case its expression in C1 could be better explained by tumor type than by tumor purity (Fig. 2C). The final pool used for the selection of signature genes included 9950 genes (Fig. 2D).

Next, genes with subtype-specific expression were identified by analysis of variance. 5778 genes showed significant variation between subtypes after correction for multiple testing using Holm's method. Subsequent pairwise comparisons between subtypes by Tukey's test revealed 1442 genes to be specifically expressed in ccRCC, 1349 in chRCC, and 841 in pRCC. Median expression per subtype was determined for the 3632 candidate genes. The minimum log fold change compared to the two respective other entities was calculated using the absolute values of the log fold changes (Fig. 2E). Genes were ordered by decreasing log fold change per subtype and expression values were transformed to linear space.

## Selection of the signature matrix

From the set of 3632 tumor-specific genes a subset was extracted that efficiently, with respect to the number of included genes, uncovers the proportional composition of characteristics from ccRCC, chRCC and pRCC in RCC samples. Besides distinguishing between histologically distinct ccRCC, chRCC or pRCC cases, it should be able to identify heterogeneous tumors.

As in similar studies (A.R. Abbas et al., *loc. cit.*, A.M. Newman et al., *loc. cit.*, T. Gong et al., Optimal deconvolution of transcriptional profiling data using quadratic programming with application to complex clinical blood samples. PloS One 6(11), p. e27156 (2011), various signature matrices were created and compared (Fig. 4). The top $n$ genes with the highest log fold change per subtype were combined into a signature matrix $S_n$, i.e. $S_n$ included $3 \times n$ different genes. Each matrix $S_n$ was used to perform a subtype prediction in cohort C2 (Fig. 3). $n$ was iterated from 5 to 500 and for $n > 5$ the difference in the subtype assignments between two consecutive signature matrices $S_n$ and $S_{n-1}$ were calculated for each sample. The maximum absolute difference (MAD) between two assignments for a sample, i.e. $max(|c_n - c_{n-1}|, |p_n - p_{n-1}|, |hn - h_{n-1}|)$, was used for this.

The condition number allows measuring the sensitivity of a matrix to changes or errors in the input. Here, the

condition number, $K_n$, was calculated for each signature matrix using the R basic function kappa. Figure 4A shows the maximum MAD that was reached by one sample between consecutive signature matrices and the condition numbers $K_n$ of the analyzed matrices. For example in (A.R. Abbas et al., *loc. cit.*; A.M. Newman et al., *loc. cit.,* T. Gong et al., *loc. cit.*) the matrix with the smallest condition number was selected, since it has been shown that sensitivity of the matrix is related to accuracy of the generated predictions (A.R. Abbas et al., *loc. cit.*; T. Gong et al., *loc. cit.*). The matrix with the smallest **K** was reached here already for **$n$ = 12.** However, subsequent matrices with **$n$ > 12** still led to substantial variability in PSA (Fig. 4A). Therefore, the final signature matrix was determined here as follows: Based on the assumption that more involved genes allow for a more precise estimation, the largest matrix was chosen, that led to a relevant MAD in the estimation (MAD > 5%) for a substantial portion of C2. To increase stability of decision basis, different cohort compositions were simulated by subset sampling. In total 10000 times 50% subsets were randomly drawn from C2. Figure 4B shows for each matrix $S_n$ the proportions of the sampled subsets that experienced a MAD > 5% compared to the previous matrix. $S_{102}$, referred to as *RCC102* in the main text, was the largest matrix significantly modifying a substantial portion of the samples relative to the predecessor matrix (on average 10% per sampled subset) and therefore has been chosen as signature matrix.

3. Results

Definition of gene signature matrix for deconvolution of RCC

Using the 52 RCC from cohort C1 comprising 18 ccRCC, 16 chRCC and 18 pRCC cases, each of which could be uniquely assigned by two independent teams of pathologists, candidates for the gene signature matrix were determined (Fig. 2). To be shortlisted genes needed to be present in TCGA RNA-Seq data as well as in Affymetrix platforms HTA 2.0 and HG U133 Plus 2.0. Further, median expression had to be above the global median expression in at least one subtype. By means of TCGA RCC cohorts K. Yoshihara et al. *loc. cit.* have shown that tumor purity explicitly varies between RCC subtypes (Fig. 2B). The signature to be established should be independent of tumor purity to be able to classify beside primary tumor tissue homogeneous tumor cells as well. Therefore, genes more related to tumor purity, as determined by the ESTIMATE method (K. Yoshihara et al. *loc. cit.*), than to tumor type were excluded (Fig. 2C).

9950 genes remained after filtering and were tested on differential expression between RCC subtypes by analysis of variance (Fig. 2D). Subsequent post-hoc testing using Tukey's method revealed 1442 genes to be specifically expressed in ccRCC, 1349 in chRCC, and 841 in pRCC (Fig. 2E). From this set of specifically expressed genes signature genes have been selected by evaluating various signature gene matrices. Starting with the genes exhibiting highest log fold change compared to the two respective other subtypes iteratively matrices with increasing number of genes were created and applied to deconvolve the 143 samples from cohort C2 (Fig. 3). Robust linear regression was employed for deconvolution. Matrices consisted of median expression values per RCC subtype calculated from C1. Matrix sizes ranged from 15, i.e. top five genes per subtype, to 1500 genes (Fig. 4A). Since RCC samples with defined histological composition were not available supervised learning approaches could not be used to define the optimal signature. Therefore, a heuristic criterion was applied: Based on the assumption that resolution of deconvolution increases with the number of involved genes, the largest matrix achieving substantial change in subtype deconvolution compared to its predecessor matrix was chosen. If assigned proportional composition differed by more than 5% for a sample between consecutive matrices the change was considered substantial. Different cohort compositions were simulated by 50% subset sampling from C2 and the fraction of samples affected by substantial change was determined for each tested matrix. In this way, matrix *RCC102* including the top 102 genes per RCC subtype was chosen as final signature (Fig. 4B).

Table 1 lists the top 102 genes for determining the pRCC subtype, Table 2 lists the top 102 genes for determining the ccRCC subtype, and Table 3 lists the top 102 genes for determining the chRCC subtype. 'GeneID' refers to the identifier that is assigned to a gene record in the 'Entrez Gene' database. The 'Symbol' column lists the HUGO Gene symbol of the genes. The columns headlined 'ccRCC', 'chRCC', and 'pRCC' list median expression values of the respective signature genes in the indicated RCC subtypes. The expression values are (unlogtransformed) processed signal intensities measured with the Affymetrix HTA2.0 array.

Table 1

| pRCC-specifc | | | | |
|---|---|---|---|---|
| GeneID | Symbol | ccRCC | chRCC | pRCC |
| 6590 | SLPI | 64 | 71 | 1172 |
| 10568 | SLC34A2 | 98 | 59 | 1325 |
| 5649 | RELN | 24 | 26 | 318 |
| 164312 | LRRN4 | 38 | 40 | 488 |

(continued)

| pRCC-specifc | | | | |
|---|---|---|---|---|
| GeneID | Symbol | ccRCC | chRCC | pRCC |
| 5284 | PIGR | 73 | 152 | 1668 |
| 8842 | PROM1 | 33 | 39 | 387 |
| 27255 | CNTN6 | 12 | 14 | 125 |
| 80736 | SLC44A4 | 95 | 72 | 791 |
| 1365 | CLDN3 | 223 | 236 | 1480 |
| 7348 | UPK1B | 24 | 25 | 137 |
| 4316 | MMP7 | 149 | 78 | 808 |
| 10103 | TSPAN1 | 152 | 166 | 738 |
| 5950 | RBP4 | 68 | 69 | 298 |
| 6372 | CXCL6 | 18 | 19 | 72 |
| 346389 | MACC1 | 141 | 75 | 485 |
| 83543 | AIF1L | 98 | 69 | 323 |
| 3595 | IL12RB2 | 32 | 28 | 100 |
| 135932 | TMEM139 | 84 | 85 | 257 |
| 25805 | BAMBI | 79 | 71 | 239 |
| 2239 | GPC4 | 113 | 74 | 338 |
| 3880 | KRT19 | 141 | 78 | 421 |
| 3918 | LAMC2 | 53 | 60 | 178 |
| 5789 | PTPRD | 66 | 80 | 234 |
| 6662 | SOX9 | 172 | 121 | 490 |
| 63917 | GALNT11 | 155 | 153 | 436 |
| 54716 | SLC6A20 | 57 | 77 | 212 |
| 30811 | HUNK | 56 | 46 | 153 |
| 169693 | TMEM252 | 43 | 33 | 116 |
| 144165 | PRICKLE1 | 65 | 61 | 169 |
| 2296 | FOXC1 | 83 | 68 | 217 |
| 8741 | TNFSF13 | 160 | 152 | 402 |
| 6581 | SLC22A3 | 39 | 34 | 98 |
| 6659 | SOX4 | 178 | 140 | 447 |
| 26022 | TMEM98 | 130 | 121 | 325 |
| 166647 | ADGRA3 | 98 | 82 | 235 |
| 27445 | PCLO | 48 | 57 | 133 |
| 54825 | CDHR2 | 63 | 65 | 152 |
| 51435 | SCARA3 | 126 | 102 | 280 |
| 3912 | LAMB1 | 320 | 380 | 841 |
| 5076 | PAX2 | 168 | 146 | 371 |
| 94234 | FOXQ1 | 78 | 78 | 171 |
| 54756 | IL17RD | 53 | 55 | 120 |
| 222236 | NAPEPLD | 72 | 61 | 153 |
| 1645 | AKR1C1 | 71 | 113 | 239 |
| 7474 | WNT5A | 42 | 41 | 88 |
| 4232 | MEST | 53 | 50 | 111 |
| 6565 | SLC15A2 | 35 | 28 | 72 |
| 80162 | PGGHG | 113 | 108 | 232 |
| 677819 | SNORA37 | 409 | 406 | 832 |
| 729438 | CASTOR2 | 102 | 84 | 206 |
| 126969 | SLC44A3 | 108 | 98 | 215 |
| 8612 | PLPP2 | 111 | 109 | 222 |

(continued)

| pRCC-specifc | | | | |
|---|---|---|---|---|
| GeneID | Symbol | ccRCC | chRCC | pRCC |
| 221061 | FAM171A1 | 103 | 134 | 265 |
| 387758 | FIBIN | 55 | 52 | 109 |
| 23007 | PLCH1 | 46 | 53 | 103 |
| 51330 | TNFRSF12A | 641 | 601 | 1242 |
| 3489 | IGFBP6 | 84 | 85 | 164 |
| 1523 | CUX1 | 214 | 185 | 414 |
| 4634 | MYL3 | 69 | 73 | 141 |
| 23359 | FAM189A1 | 53 | 59 | 112 |
| 56245 | C21orf62 | 43 | 46 | 87 |
| 5745 | PTH1R | 96 | 90 | 181 |
| 10749 | KIF1C | 101 | 93 | 190 |
| 113026 | PLCD3 | 96 | 94 | 179 |
| 114884 | OSBPL10 | 100 | 75 | 185 |
| 6608 | SMO | 116 | 114 | 215 |
| 9476 | NAPSA | 74 | 68 | 137 |
| 2937 | GSS | 146 | 149 | 275 |
| 400566 | C17orf97 | 44 | 52 | 96 |
| 2314 | FLII | 214 | 198 | 390 |
| 274 | BIN1 | 158 | 143 | 284 |
| 2121 | EVC | 169 | 144 | 303 |
| 81615 | TMEM163 | 58 | 63 | 113 |
| 11030 | RBPMS | 269 | 219 | 483 |
| 4214 | MAP3K1 | 234 | 173 | 417 |
| 341 | APOC1 | 87 | 72 | 155 |
| 3200 | HOXA3 | 88 | 95 | 169 |
| 54997 | TESC | 71 | 73 | 129 |
| 2036 | EPB41 L1 | 128 | 145 | 252 |
| 58485 | TRAPPC1 | 298 | 285 | 519 |
| 348801 | LNP1 | 39 | 38 | 68 |
| 283373 | ANKRD52 | 122 | 118 | 212 |
| 131566 | DCBLD2 | 178 | 131 | 306 |
| 55349 | CHDH | 85 | 80 | 147 |
| 26470 | SEZ6L2 | 134 | 97 | 231 |
| 113220 | KIF12 | 120 | 117 | 204 |
| 112817 | HOGA1 | 60 | 62 | 105 |
| 23326 | USP22 | 395 | 375 | 675 |
| 91316 | GUSBP11 | 166 | 154 | 283 |
| 64129 | TINAGL1 | 149 | 173 | 293 |
| 79541 | OR2A4 | 48 | 59 | 98 |
| 367 | AR | 59 | 36 | 98 |
| 64084 | CLSTN2 | 49 | 57 | 94 |
| 84662 | GLIS2 | 240 | 280 | 459 |
| 10140 | TOB1 | 131 | 115 | 214 |
| 9209 | LRRFIP2 | 67 | 55 | 110 |
| 164091 | PAQR7 | 69 | 66 | 111 |
| 89932 | PAPLN | 110 | 108 | 177 |
| 10610 | ST6GALNAC2 | 53 | 54 | 87 |
| 7703 | PCGF2 | 154 | 154 | 247 |

(continued)

| pRCC-specifc | | | | |
|---|---|---|---|---|
| GeneID | Symbol | ccRCC | chRCC | pRCC |
| 5493 | PPL | 86 | 97 | 154 |
| 342527 | SMTNL2 | 98 | 97 | 156 |

Table 2

| ccRCC-specifc | | | | |
|---|---|---|---|---|
| GeneID | Symbol | ccRCC | chRCC | pRCC |
| 1356 | CP | 726 | 14 | 15 |
| 4015 | LOX | 909 | 43 | 42 |
| 2938 | GSTA1 | 246 | 17 | 17 |
| 1573 | CYP2J2 | 224 | 20 | 23 |
| 51129 | ANGPTL4 | 696 | 70 | 73 |
| 5350 | PLN | 174 | 19 | 18 |
| 112399 | EGLN3 | 694 | 57 | 80 |
| 29974 | A1CF | 147 | 13 | 18 |
| 1038 | CDR1 | 313 | 30 | 39 |
| 2487 | FRZB | 504 | 59 | 66 |
| 10050 | SLC17A4 | 242 | 22 | 34 |
| 1244 | ABCC2 | 158 | 21 | 22 |
| 6515 | SLC2A3 | 1001 | 111 | 142 |
| 29923 | HILPDA | 460 | 51 | 66 |
| 115361 | GBP4 | 404 | 65 | 52 |
| 23236 | PLCB1 | 302 | 47 | 52 |
| 4311 | MME | 191 | 30 | 33 |
| 2327 | FMO2 | 183 | 19 | 32 |
| 10203 | CALCRL | 292 | 51 | 51 |
| 2157 | F8 | 275 | 49 | 43 |
| 9457 | FHL5 | 128 | 24 | 18 |
| 8564 | KMO | 84 | 12 | 16 |
| 80243 | PREX2 | 158 | 31 | 20 |
| 5166 | PDK4 | 492 | 79 | 101 |
| 3625 | INHBB | 239 | 53 | 42 |
| 159963 | SLC5A12 | 68 | 15 | 15 |
| 5054 | SERPINE1 | 372 | 84 | 81 |
| 6513 | SLC2A1 | 589 | 133 | 135 |
| 3486 | IGFBP3 | 1433 | 336 | 345 |
| 2113 | ETS1 | 392 | 80 | 96 |
| 5033 | P4HA1 | 669 | 150 | 166 |
| 1009 | CDH11 | 228 | 57 | 60 |
| 57493 | HEG1 | 510 | 139 | 129 |
| 3678 | ITGA5 | 399 | 109 | 110 |
| 57561 | ARRDC3 | 932 | 215 | 259 |
| 10186 | LHFPL6 | 703 | 209 | 159 |
| 230 | ALDOC | 232 | 70 | 65 |
| 6925 | TCF4 | 294 | 88 | 82 |
| 8516 | ITGA8 | 146 | 44 | 35 |
| 168537 | GIMAP7 | 422 | 127 | 101 |

(continued)

| ccRCC-specifc | | | | |
|---|---|---|---|---|
| **GeneID** | **Symbol** | **ccRCC** | **chRCC** | **pRCC** |
| 83872 | HMCN1 | 96 | 29 | 23 |
| 5163 | PDK1 | 308 | 93 | 87 |
| 2 | A2M | 1675 | 511 | 459 |
| 23516 | SLC39A14 | 404 | 115 | 125 |
| 5139 | PDE3A | 83 | 26 | 20 |
| 55076 | TMEM45A | 86 | 25 | 27 |
| 5352 | PLOD2 | 722 | 203 | 225 |
| 1843 | DUSP1 | 868 | 271 | 217 |
| 3910 | LAMA4 | 400 | 126 | 71 |
| 664 | BNIP3 | 690 | 185 | 219 |
| 80310 | PDGFD | 344 | 76 | 111 |
| 10964 | IFI44L | 129 | 23 | 42 |
| 4360 | MRC1 | 174 | 57 | 36 |
| 768 | CA9 | 182 | 61 | 60 |
| 50937 | CDON | 164 | 56 | 56 |
| 1903 | S1PR3 | 193 | 66 | 62 |
| 1363 | CPE | 276 | 95 | 93 |
| 9535 | GMFG | 439 | 137 | 151 |
| 11067 | DEPP1 | 348 | 120 | 91 |
| 5256 | PHKA2 | 363 | 122 | 126 |
| 9844 | ELMO1 | 183 | 55 | 64 |
| 1282 | COL4A1 | 497 | 169 | 173 |
| 1193 | CLIC2 | 121 | 42 | 41 |
| 26577 | PCOLCE2 | 75 | 26 | 26 |
| 23491 | CES3 | 175 | 61 | 62 |
| 56901 | NDUFA4L2 | 303 | 108 | 94 |
| 6518 | SLC2A5 | 237 | 62 | 85 |
| 2628 | GATM | 79 | 23 | 29 |
| 84251 | SGIP1 | 86 | 28 | 31 |
| 3760 | KCNJ3 | 102 | 25 | 37 |
| 2013 | EMP2 | 306 | 114 | 101 |
| 400759 | GBP1P1 | 88 | 30 | 33 |
| 1730 | DIAPH2 | 250 | 84 | 96 |
| 2022 | ENG | 735 | 280 | 286 |
| 4208 | MEF2C | 160 | 63 | 58 |
| 1528 | CYB5A | 879 | 347 | 321 |
| 79644 | SRD5A3 | 352 | 121 | 139 |
| 51141 | INSIG2 | 392 | 156 | 134 |
| 6678 | SPARC | 2447 | 671 | 974 |
| 51203 | NUSAP1 | 87 | 35 | 34 |
| 2114 | ETS2 | 477 | 193 | 191 |
| 4921 | DDR2 | 95 | 39 | 30 |
| 9934 | P2RY14 | 67 | 28 | 25 |
| 7145 | TNS1 | 540 | 208 | 224 |
| 1615 | DARS | 391 | 159 | 163 |
| 10008 | KCNE3 | 180 | 62 | 76 |
| 7070 | THY1 | 354 | 150 | 125 |
| 7025 | NR2F1 | 154 | 65 | 62 |

(continued)

| ccRCC-specifc | | | | |
|---|---|---|---|---|
| GeneID | Symbol | ccRCC | chRCC | pRCC |
| 1910 | EDNRB | 592 | 148 | 252 |
| 6472 | SHMT2 | 452 | 193 | 177 |
| 84154 | RPF2 | 343 | 146 | 130 |
| 1809 | DPYSL3 | 131 | 56 | 47 |
| 4734 | NEDD4 | 129 | 41 | 55 |
| 441478 | NRARP | 115 | 50 | 35 |
| 8291 | DYSF | 159 | 68 | 59 |
| 56925 | LXN | 111 | 48 | 46 |
| 257019 | FRMD3 | 124 | 44 | 54 |
| 3176 | HNMT | 149 | 56 | 65 |
| 4601 | MXI1 | 301 | 114 | 131 |
| 51365 | PLA1A | 199 | 87 | 59 |
| 11149 | BVES | 78 | 34 | 31 |
| 3939 | LDHA | 1994 | 881 | 691 |

Table 3

| chRCC-specifc | | | | |
|---|---|---|---|---|
| GeneID | Symbol | ccRCC | chRCC | pRCC |
| 26228 | STAP1 | 11 | 1088 | 11 |
| 245972 | ATP6V0D2 | 25 | 2454 | 30 |
| 253012 | HEPACAM2 | 9 | 534 | 10 |
| 116449 | CLNK | 18 | 809 | 19 |
| 121506 | ERP27 | 69 | 2865 | 63 |
| 5816 | PVALB | 26 | 939 | 31 |
| 9073 | CLDN8 | 7 | 239 | 8 |
| 51458 | RHCG | 43 | 1295 | 48 |
| 2299 | FOXI1 | 32 | 846 | 38 |
| 127124 | ATP6V1G3 | 6 | 130 | 6 |
| 245973 | ATP6V1C2 | 28 | 544 | 29 |
| 158401 | SHOC1 | 8 | 133 | 9 |
| 658 | BMPR1B | 18 | 255 | 18 |
| 200958 | MUC20 | 224 | 2789 | 150 |
| 6521 | SLC4A1 | 59 | 834 | 69 |
| 120939 | TMEM52B | 35 | 476 | 41 |
| 1950 | EGF | 36 | 394 | 29 |
| 222545 | GPRC6A | 10 | 112 | 11 |
| 155006 | TMEM213 | 30 | 406 | 40 |
| 57830 | KRTAP5-8 | 56 | 632 | 65 |
| 27199 | OXGR1 | 15 | 163 | 17 |
| 80157 | CWH43 | 20 | 209 | 22 |
| 2888 | GRB14 | 51 | 524 | 55 |
| 3816 | KLK1 | 46 | 484 | 56 |
| 1080 | CFTR | 19 | 187 | 22 |
| 10512 | SEMA3C | 79 | 665 | 62 |
| 23327 | NEDD4L | 120 | 1006 | 121 |
| 5618 | PRLR | 41 | 341 | 33 |

(continued)

| chRCC-specifc | | | | |
|---|---|---|---|---|
| GeneID | Symbol | ccRCC | chRCC | pRCC |
| 129684 | CNTNAP5 | 21 | 211 | 25 |
| 9194 | SLC16A7 | 58 | 565 | 69 |
| 266722 | HS6ST3 | 31 | 255 | 31 |
| 525 | ATP6V1B1 | 54 | 498 | 64 |
| 7809 | BSND | 34 | 272 | 38 |
| 221981 | THSD7A | 81 | 608 | 86 |
| 53828 | FXYD4 | 48 | 368 | 53 |
| 199920 | FYB2 | 12 | 85 | 12 |
| 7069 | THRSP | 40 | 310 | 45 |
| 51635 | DHRS7 | 193 | 1307 | 195 |
| 6549 | SLC9A2 | 13 | 115 | 17 |
| 7368 | UGT8 | 94 | 620 | 88 |
| 5569 | PKIA | 29 | 210 | 32 |
| 55026 | TMEM255A | 56 | 497 | 77 |
| 84803 | GPAT3 | 59 | 392 | 64 |
| 57533 | TBC1D14 | 193 | 1167 | 172 |
| 65267 | WNK3 | 20 | 120 | 20 |
| 7113 | TMPRSS2 | 37 | 303 | 51 |
| 54899 | PXK | 86 | 582 | 99 |
| 9068 | ANGPTL1 | 13 | 76 | 14 |
| 2104 | ESRRG | 37 | 279 | 51 |
| 121601 | ANO4 | 27 | 145 | 21 |
| 126868 | MAB21L3 | 31 | 165 | 31 |
| 9413 | FAM189A2 | 40 | 212 | 41 |
| 202374 | STK32A | 18 | 99 | 19 |
| 23086 | EXPH5 | 64 | 329 | 53 |
| 79611 | ACSS3 | 153 | 898 | 178 |
| 255189 | PLA2G4F | 50 | 287 | 57 |
| 8825 | LIN7A | 111 | 544 | 81 |
| 26049 | FAM169A | 16 | 85 | 17 |
| 744 | MPPED2 | 34 | 164 | 33 |
| 57687 | VAT1L | 39 | 190 | 41 |
| 89894 | TMEM116 | 70 | 374 | 82 |
| 84216 | TMEM117 | 66 | 298 | 67 |
| 26249 | KLHL3 | 38 | 176 | 40 |
| 4306 | NR3C2 | 60 | 302 | 68 |
| 760 | CA2 | 121 | 535 | 89 |
| 2620 | GAS2 | 20 | 89 | 19 |
| 6690 | SPINK1 | 40 | 175 | 30 |
| 3606 | IL18 | 114 | 575 | 132 |
| 51301 | GCNT4 | 32 | 140 | 28 |
| 57127 | RHBG | 47 | 234 | 54 |
| 29899 | GPSM2 | 47 | 253 | 59 |
| 55605 | KIF21A | 158 | 721 | 168 |
| 92597 | MOB1B | 95 | 405 | 74 |
| 10655 | DMRT2 | 43 | 213 | 50 |
| 6785 | ELOVL4 | 24 | 100 | 23 |
| 26499 | PLEK2 | 107 | 449 | 106 |

(continued)

| chRCC-specifc | | | | |
|---|---|---|---|---|
| GeneID | Symbol | ccRCC | chRCC | pRCC |
| 5019 | OXCT1 | 76 | 351 | 84 |
| 6641 | SNTB1 | 143 | 597 | 125 |
| 64409 | GALNT17 | 38 | 192 | 46 |
| 134111 | UBE2QL1 | 35 | 140 | 31 |
| 6309 | SC5D | 104 | 419 | 90 |
| 5580 | PRKCD | 150 | 594 | 147 |
| 5874 | RAB27B | 36 | 140 | 33 |
| 3419 | IDH3A | 216 | 839 | 215 |
| 25769 | SLC24A2 | 19 | 85 | 22 |
| 1773 | DNASE1 | 57 | 268 | 70 |
| 38 | ACAT1 | 225 | 1194 | 311 |
| 158326 | FREM1 | 26 | 142 | 37 |
| 23303 | KIF13B | 150 | 760 | 198 |
| 100505835 | LINC01532 | 51 | 205 | 54 |
| 3418 | IDH2 | 319 | 1193 | 281 |
| 25825 | BACE2 | 124 | 635 | 171 |
| 6653 | SORL1 | 258 | 955 | 211 |
| 92949 | ADAMTSL1 | 33 | 124 | 34 |
| 9331 | B4GALT6 | 46 | 168 | 42 |
| 11057 | ABHD2 | 356 | 1296 | 354 |
| 5255 | PHKA1 | 51 | 183 | 43 |
| 2066 | ERBB4 | 23 | 88 | 24 |
| 1717 | DHCR7 | 133 | 598 | 167 |
| 272 | AMPD3 | 86 | 305 | 81 |
| 164832 | LONRF2 | 33 | 114 | 33 |
| 23382 | AHCYL2 | 85 | 387 | 111 |

Deconvolution of the TCGA RCC cohort

*RCC102* was used to perform proportional subtype assignment (PSA) by deconvolution of 864 tumor transcriptomes from the combined TCGA RCC cohort, including the KIRC, KIRP, and KICH cohorts. Additionally, using the 306 signature genes from *RCC102* a Principal Component Analysis (PCA) was carried out with the TCGA cohort (Fig. 9A). Here, tumors mostly grouped according to their original classification. Principal component 1 discriminated tumors originating from distal cell types (KICH) from those arising from the proximal tubule (KIRC, KIRP). The incorrect classification of some TCGA samples, in particular the wrong assignment of some KICH samples, has been reported in detail elsewhere (F. Büttner et al., Survival Prediction of Clear Cell Renal Cell Carcinoma Based on Gene Expression Similarity to the Proximal Tubule of the Nephron. Eur. Urol. 68(6), p. 1016-1020 (2015); C.J. Ricketts et al., *loc. cit.*) and was again obvious here. Interestingly, KIRC and KIRP cohorts were not fully separable from each other by PC1 and PC2. In Figure 5 only those samples were colored that were estimated by PSA to represent a single subtype to at least 95%. Using this threshold, about 30% (n=256) of the tumors could not be assigned to one subtype. Further, an empirical P-value was determined representing the probability that the signature cannot be used to estimate the subtype composition of a sample (black colored samples in Figure 4 had P-value > 0.05).

Prognostic classification of RCC based on PSA

RCC subtypes vary in prognosis (C.J. Ricketts et al., *loc. cit.*) (Fig. 6A). Hence, the inventors were wondering if PSA produced by deconvolution was predictive of patient survival as well. Using conditional inference trees with endpoint cancer-specific survival (CSS) the TCGA RCC cohort could be split in three patient groups with significantly varying survival probabilities (Fig. 7). The risk groups were termed "good", "intermediate", and "poor" according to their prognosis (Fig. 10B/C). Next, the inventors analyzed how these groups were related to the original (histological) classification of the TCGA RCC cohort (Fig. 10D). The "good"-group containing tumors with ccRCC proportion $c < 13.7\%$ included almost the entire KICH cohort and the majority of the KIRP cohort. Depending on the pRCC content $p$ the remaining tumors were either sorted into the "intermediate"-group ($p < 16.5\%$) or the "poor"-group ($p > 16.5\%$). KIRC cases made up 98% of the tumors in the "intermediate"-group. The "poor"-group comprised tumors exhibiting both increased ccRCC content and increased pRCC content and thus included almost exclusively samples from the KIRP and the KIRC cohort.

The ranges for the survival likelihood after 5 years can be represented by the confidence intervals of the respective groups. The confidence intervals for each group were determined as being as follows. Entire cohort: 79% (CI: 75% - 82%); good: 92% (CI: 87% - 96%); intermediate: 77% (CI: 72% - 82%); poor: 49% (CI: 37% - 65%).

Ricketts et al. *loc cit.* recently published an updated histological classification of the TCGA RCC cohort. In particular, the heterogeneity of papillary RCC had been addressed in the new classification; the KIRP cohort was subdivided in the CpG island methylator phenotype-associated (CIMP)-RCC, pRCC type 1 and 2, and unclassified pRCC (Fig. 6). The different pRCC types differed in prognosis with CIMP having the poorest and pRCC type 1 the best survival probabilities (Fig. 6A). Appropriately, all CIMP cases were grouped to the "poor"-group by PSA, while 96% of pRCC type 1 were sorted into the "good"-group. Interestingly, pRCC type 2 was split almost equally between "good" (52%) and "poor" (45%). Comparing the prognostic value of PSA-based risk groups and histological classification revealed that both provide independent information, however, partitioning based on PSA clearly outperformed the histological classification (Fig. 9F).

4. Association to survival of proportional subtype assignments using different signature gene subsets

A question that arose was whether subsets of the 3 x 102 (=306) signature genes are already sufficient for determining in a subject's biological sample the relative proportions of ccRCC, pRCC, and chRCC. The inventors proceeded as follows: The 306 genes are composed of the 102 top-specific genes per subtype. Now, starting with the top 2 genes per subtype (i.e. 6 genes in total), the inventors performed the group definition procedure (see tree structure) and subsequent survival time analysis. For this the inventors used the TCGA cohort (n=836). This procedure was repeated for the top 3 up to the top 102 genes. Interestingly, for each of the 101 tested signature matrices a (significant) division into subgroups can be found. What is decisive is that each of the 101 subgroups tested has a significant connection with survival. The P-values from the survival time analyses are shown in the attached Figure 8.

Computational gene expression deconvolution is performed by solving a linear system using regression methods such as least square regression, support vector regression, or preferably robust linear regression. In order to derive estimates of the three proportions (pRCC, ccRCC, and chRCC) at least three equations in the linear system are necessary, corresponding to three genes in the signature matrix. In case of three genes in the signature matrix, a sufficient condition for the linear system to have a solution is that these equations (i.e. the rows of the matrix) are linear independent. In our method, this condition can be satisfied by an appropriate selection of three genes, each specific in exactly one of the subtypes.

As a consequence, even with only one gene per subgroup type, i.e. from each of Table 1, Table 2, and Table 3, preferably with two genes per subgroup type a reliable subtype classification can be made.

5. Clustering a RCC cohort by using principal component analysis (PCA)

The inventors tested whether signal separation methods other than deconvolution can be used to carry out the invention on the basis of the 3 x 102 (=306) signature genes. Deconvolution makes it possible to analyze individual samples; a forecast is then made on the basis of the relative proportions. Alternatively, the 306 genes can be used to cluster a comprehensive RCC cohort or make a principal component analysis (PCA). The clustering obtained could then be used as a reference for new, unknown samples: One measures the 306 genes in the new sample and clusters them together with the reference cohort and looks into which cluster the new sample falls. The attached Figure 10 depicting a PCA plot illustrates this. It shows the result of a PCA with the TCGA cohort based on the 306 signature genes according to the invention. The samples are colored according to their risk group. One can see that the risk groups can be separated.

6. Implementation of the invention into daily clinical routine

Tissue (either fresh, fresh-frozen or FFPE) or body fluids like blood plasma or urine of a patient with RCC is obtained. It may be subjected to the hospital's laboratory or delivered from the hospital or out-patient center to a specialized laboratory. Nucleic acids (total RNA) will be prepared by standard methods. Quantification of the expression levels of candidate genes will be performed using state-of-the art methods. Here different methods like RNA sequencing, microarray or chip based technology or RT-PCR etc. can be used. Based on the established gene signature, (deconvolution-) analysis using well-established algorithms (e.g. robust linear regression) will be performed to determine the proportions of ccRCC, pRCC and chRCC in the sample, subsequently resulting in the outcome classification of RCC patients (good, intermediate, poor outcome). This report will be delivered to the respective physician, who requested the analyses of RCC specimen.

7. Example for making a diagnosis by the method according to the invention

In the following an example is provided where on the basis of two genes per RCC subgroup a diagnosis is made for a patient suffering from RCC. The tissue sample of the patient is referred to as TCGA-CZ-4857-01A-01R-1305-07.

Selection of the two top specific genes per subtype results in the reduced signature matrix **RCC2**:

| RCC2 | pRCC | ccRCC | chRCC |
|---|---|---|---|
| SLPI | 1172 | 64 | 71 |
| SLC34A2 | 1325 | 98 | 59 |
| CP | 15 | 726 | 14 |
| LOX | 42 | 909 | 43 |
| STAP1 | 11 | 11 | 1088 |
| ATP6V0D2 | 30 | 25 | 2454 |

For deconvolution, columns of *RCC*2 are centered to zero mean and scaled to unit variance, resulting in the scaled signature matrix *RCC*2_z:

| RCC2_z | pRCC | ccRCC | chRCC |
|---|---|---|---|
| SLPI | 1.1663743 | -0.6008201 | -0.556154 |
| SLC34A2 | 1.4076932 | -0.5162326 | -0.5682773 |
| CP | -0.6585007 | 1.0461484 | -0.6137395 |
| LOX | -0.615915 | 1.5014282 | -0.5844416 |
| STAP1 | -0.6648097 | -0.7326771 | 0.4712913 |
| ATP6V0D2 | -0.634842 | -0.6978469 | 1.8513211 |

PSA are calculated for sample TCGA-CZ-4857-01A-01R-1305-07 from TCGA KIRC cohort. FPKM-UQ expression values were obtained from https://portal.gdc.cancer.gov/. For the six genes in RCC2_z, the vector *m* containing these values is given by:

| m | TCGA-CZ-4857-01A-01R-1305-07 |
|---|---|
| SLPI | 560149.7781 |
| SLC34A2 | 376720.7362 |
| CP | 2347358.0815 |
| LOX | 5671940.0793 |
| STAP1 | 5467.4902 |
| ATP6V0D2 | 316.4433 |

As for the signature matrix, values in *m* are centered to zero mean and scaled to unit variance, resulting in the scaled expression profile *m_z*:

| m_z | TCGA-CZ-4857-01A-01R-1305-07 |
|---|---|
| SLPI | -0.4195759 |
| SLC34A2 | -0.5020201 |
| CP | 0.3837046 |
| LOX | 1.8779746 |
| STAP1 | -0.6688840 |
| ATP6V0D2 | -0.6711992 |

The underlying assumption of gene expression deconvolution is that expression level of gene *i* in *m* is the sum of its expression in the ccRCC, pRCC and chRCC proportions of sample TCGA-CZ-4857-01A-01R-1305-07. Vector *f* shall denote these unknown proportions of ccRCC, pRCC und chRCC in sample TCGA-CZ-4857-01A-01R-1305-07. *f* is estimated by solving the linear system *m_z = RCC2_z·f* using regression methods such as robust linear regression. The function "rim" from the R-package MASS performs a robust linear regression and is applied as follows:

$$fit = rlm(m\_z \sim RCC2\_z, maxit = 200)$$

Resulting regression coefficients are accessed via *fit$coefficients*:

| intercept | pRCC | ccRCC | chRCC |
|---|---|---|---|
| 0.10855833 | 0.04446193 | 1.10539583 | 0.02409214 |

The intercept is discarded and percentages are calculated by dividing the three estimates by their sum resulting in following predicted subtype composition of TCGA-CZ-4857-01A-01R-1305-07:

| pRCC | ccRCC | chRCC |
|---|---|---|
| 3.787379% | 94.160392% | 2.052229% |

With a ccRCC proportion above 13.7% and a pRCC proportion below 16.5%, TCGA-CZ-4857-01A-01R-1305-07 would be assigned to prognosis group "intermediate" based on cutoffs shown in Figure 7. However, cut-offs in Figure 7 have been identified based on PSA determined with signature matrix *RCC*102 and therefore, they may not be optimal for classification of *RCC2*-based assignments.

8. Conclusion and miscellaneous

The inventors provide for the very first time an objective subtype classification or a proportional subtype assignment method for RCC which provides reliable results and is easily applicable in clinical settings.

**Claims**

1. A method for determining in a subject's biological sample the relative proportions of papillary renal cell carcinoma (pRCC), clear cell renal cell carcinoma (ccRCC), and chromophobe renal cell carcinoma (chRCC), the method comprising:

(a) Assaying a biological sample to determine expression level values of

- at least one of the signature genes listed in Table 1,
- at least one of the signature genes listed in Table 2, and
- at least one of the signature genes listed in Table 3, whereby the biological sample has been provided from a subject suspected of being affected by RCC,

(b) Subjecting the obtained expression level values to a signal separation method, thereby determining relative proportions of pRCC, ccRCC, and chRCC in said biological sample.

2. The method of claim 1, **characterized in that** in step (a) said biological sample is assayed to determine expression level values of

   - at least two of the signature genes listed in Table 1,
   - at least two of the signature genes listed in Table 2, and
   - at least two of the signature genes listed in Table 3.

3. The method of claim 1 or 2, **characterized in that** the signal separation method is a blind signal separation method.

4. The method of claim 3, **characterized in that** the blind separation method is deconvolution, preferably computational deconvolution.

5. The method of any of the preceding claims, **characterized in that** after step (b) the following step is carried out:
   (c) Classifying the subject into a risk group on the basis of the relative proportions of pRCC, ccRCC, and chRCC in said biological sample.

6. The method of claim 5, **characterized in that** the risk group is selected from "good", "intermediate", and "poor" according to the prognosis for the subject.

7. The method of claim 6, **characterized in that** the "good" group is determined by a relative ccRCC proportion of about $\leq 50\%$, further preferably about $\leq 45\%$, further preferably about $40\%$, further preferably about $\leq 35\%$, further preferably about $\leq 30\%$, further preferably about $\leq 25\%$, further preferably about $\leq 20\%$, further preferably about $\leq 15\%$, and highly preferably about $< 13.7\%$.

8. The method of claim 6 or 7, **characterized in that** the "intermediate" group is determined by a relative pRCC proportion of about $\leq 50\%$, further preferably about $\leq 45\%$, further preferably about $\leq 40\%$, further preferably about $\leq 35\%$, further preferably about $\leq 30\%$, further preferably about $\leq 25\%$, further preferably about $\leq 20\%$, and highly preferably of about $\leq 16.5\%$.

9. The method of any of claims 6-8, **characterized in that** the "poor" group is determined by a relative pRCC proportion of about $\geq 5\%$, preferably about $\geq 7.5\%$, further preferably about $\geq 10\%$, further preferably about $\geq 12\%$, and highly preferably about $> 16.5\%$.

10. The method of any of the previous claims, **characterized in that** in step (a) the assaying involves the use of RNA sequencing, a PCR-based method, a microarray-based method, a hybridization-based method and/or an antibody-based method.

11. A use of

    - at least one, preferably at least two of the signature genes listed in Table 1,
    - at least one, preferably at least two of the signature genes listed in Table 2, and
    - at least one, preferably at least two of the signature genes listed in Table 3,

    for determining in a subject's biological sample the relative proportions of papillary renal cell carcinoma (pRCC), clear cell renal cell carcinoma (ccRCC), and chromophobe renal cell carcinoma (chRCC), further preferably for classifying the subject into a RCC risk group.

**Patentansprüche**

1. Verfahren zur Bestimmung der relativen Anteile von papillärem Nierenzellkarzinom (pRCC), klarzelligem Nierenzellkarzinom (ccRCC) und chromophobem Nierenzellkarzinom (chRCC) in einer biologischen Probe eines Subjekts, wobei das Verfahren Folgendes aufweist:

   (a) Untersuchen der biologischen Probe zur Bestimmung der Expressionsspiegelwerte von

- zumindest einem der in Tabelle 1 aufgelisteten Signaturgene,
- zumindest einem der in Tabelle 2 aufgelisteten Signaturgene, und
- zumindest einem der in Tabelle 3 aufgelisteten Signaturgene,

wobei die biologische Probe von einem Subjekt stammt, bei dem der Verdacht besteht, dass es von RCC betroffen ist,

(b) Unterziehen der erhaltenen Expressionsspiegelwerte einem Signaltrennungsverfahren, wodurch relative Anteile von pRCC, ccRCC und chRCC in der biologischen Probe bestimmt werden.

2. Verfahren nach Anspruch 1, **dadurch gekennzeichnet, dass** in Schritt (a) die biologische Probe untersucht wird, um die Expressionswerte von

- zumindestens zwei der in Tabelle 1 aufgelisteten Signaturgene,
- zumindestens zwei der in Tabelle 2 aufgelisteten Signaturgene und
- zumindestens zwei der in Tabelle 3 aufgelisteten Signaturgene zu bestimmen.

3. Verfahren nach Anspruch 1 oder 2, **dadurch gekennzeichnet, dass** das Signaltrennungsverfahren ein Blindsignal-Trennungsverfahren ist.

4. Verfahren nach Anspruch 3, **dadurch gekennzeichnet, dass** es sich bei dem Blindsignal-Trennungsverfahren um eine Dekonvolution, vorzugsweise eine rechnerische Dekonvolution, handelt.

5. Verfahren nach einem der vorhergehenden Ansprüche, **dadurch gekennzeichnet, dass** nach Schritt (b) der folgende Schritt durchgeführt wird:

(c) Einteilung des Subjekts in eine Risikogruppe auf der Grundlage der relativen Anteile von pRCC, ccRCC und chRCC in der biologischen Probe.

6. Verfahren nach Anspruch 5, **dadurch gekennzeichnet, dass** die Risikogruppe entsprechend der Prognose für das Subjekt aus "gut", "mittel" und "schlecht" ausgewählt wird.

7. Verfahren nach Anspruch 6, **dadurch gekennzeichnet, dass** die "gute" Gruppe durch einen relativen ccRCC-Anteil von etwa $\leq 50$ %, weiter bevorzugt etwa $\leq 45$ %, weiter bevorzugt etwa 40 %, weiter bevorzugt etwa $\leq 35$ %, weiter bevorzugt etwa $\leq 30$ %, weiter bevorzugt etwa $\leq 25$ %, weiter bevorzugt etwa $\leq 20$ %, weiter bevorzugt etwa $\leq 15$ % und höchst bevorzugt etwa < 13,7 % bestimmt wird.

8. Verfahren nach Anspruch 6 oder 7, **dadurch gekennzeichnet, dass** die "mittlere" Gruppe durch einen relativen pRCC-Anteil von etwa $\leq 50$ %, weiter bevorzugt etwa $\leq 45$ %, weiter bevorzugt etwa $\leq 40$ %, weiter bevorzugt etwa $\leq 35$ %, weiter bevorzugt etwa $\leq 30$ %, weiter bevorzugt etwa $\leq 25$ %, weiter bevorzugt etwa $\leq 20$ % und höchst bevorzugt von etwa $\leq 16,5$ % bestimmt wird.

9. Verfahren nach einem der Ansprüche 6 bis 8, **dadurch gekennzeichnet, dass** die "schlechte" Gruppe durch einen relativen pRCC-Anteil von etwa $\geq 5$ %, bevorzugt etwa $\geq 7,5$ %, weiter bevorzugt etwa $\geq 10$ %, weiter bevorzugt etwa $\geq 12$ % und höchst bevorzugt etwa > 16,5 % bestimmt wird.

10. Verfahren nach einem der vorhergehenden Ansprüche, **dadurch gekennzeichnet, dass** in Schritt (a) das Untersuchen die Verwendung einer RNA-Sequenzierung, eines PCR-basierten Verfahrens, eines Microarray-basierten Verfahrens, eines Hybridisierungs-basierten Verfahrens und/oder eines Antikörper-basierten Verfahrens beinhaltet.

11. Verwendung von

- zumindest einem, vorzugsweise zumindest zwei der in Tabelle 1 aufgelisteten Signaturgenen,
- zumindest einem, vorzugsweise zumindest zwei der in Tabelle 2 aufgelisteten Signaturgenen, und
- zumindest einem, vorzugsweise zumindest zwei der in Tabelle 3 aufgelisteten Signaturgenen,

zur Bestimmung der relativen Anteile des papillären Nierenzellkarzinoms (pRCC), des klarzelligen Nierenzellkarzinoms (ccRCC) und des chromophoben Nierenzellkarzinoms (chRCC) in einer biologischen Probe eines Subjekts, ferner vorzugsweise zur Klassifizierung des Subjekts in eine RCC-Risikogruppe.

**Revendications**

1. Procédé pour déterminer dans un échantillon biologique d'un sujet les proportions relatives de carcinome papillaire à cellules rénales (pRCC), de carcinome rénal à cellules claires (ccRCC), et de carcinome chromophobe rénal (chRCC), le procédé comprenant :

   (a) l'analyse d'un échantillon biologique pour déterminer les valeurs du niveau d'expression de

   - au moins un des gènes signature listés dans le Tableau 1,
   - au moins un des gènes signature listés dans le Tableau 2, et
   - au moins un des gènes signature listés dans le Tableau 3,

   moyennant quoi l'échantillon biologique a été fourni par un sujet suspecté d'être atteint de RCC,
   (b) la soumission des valeurs du niveau d'expression obtenues à un procédé de séparation de signaux, déterminant ainsi les proportions relatives de pRCC, ccRCC et chRCC dans ledit échantillon biologique.

2. Procédé de la revendication 1, **caractérisé en ce qu'**à l'étape (a) ledit échantillon biologique est analysé pour déterminer les valeurs du niveau d'expression de

   - au moins deux des gènes signature listés dans le Tableau 1,
   - au moins deux des gènes signature listés dans le Tableau 2, et
   - au moins deux des gènes signature listés dans le Tableau 3.

3. Procédé de la revendication 1 ou 2, **caractérisé en ce que** le procédé de séparation de signaux est un procédé de séparation aveugle de signaux.

4. Procédé de la revendication 3, **caractérisé en ce que** le procédé de séparation aveugle est une déconvolution, de préférence une déconvolution informatique.

5. Procédé de l'une des revendications précédentes, **caractérisé en ce qu'**après l'étape (b), l'étape suivante est effectuée :
   (c) le classement du sujet dans un groupe à risque sur la base des proportions relatives de pRCC, ccRCC et chRCC dans ledit échantillon biologique.

6. Procédé de la revendication 5, **caractérisé en ce que** le groupe à risque est choisi parmi « bon », « intermédiaire » et « mauvais » en fonction du pronostic du sujet.

7. Procédé de la revendication 6, **caractérisé en ce que** le groupe « bon » est déterminé par une proportion relative de ccRCC d'environ $\leq 50\%$, de préférence encore d'environ $\leq 45\%$, de préférence encore d'environ 40%, de préférence encore d'environ $\leq 35\%$, de préférence encore d'environ $\leq 30\%$, de préférence encore d'environ $\leq 25\%$, de préférence encore d'environ $\leq 20\%$, de préférence encore d'environ $\leq 15\%$, et de manière hautement préférée d'environ $< 13,7\%$.

8. Procédé de la revendication 6 ou 7, **caractérisé en ce que** le groupe « intermédiaire » est déterminé par une proportion relative de pRCC d'environ $\leq 50\%$, de préférence encore d'environ $\leq 45\%$, de préférence encore d'environ $\leq 40\%$, de préférence encore d'environ $\leq 35\%$, de préférence encore d'environ $\leq 30\%$, de préférence encore d'environ $\leq 25\%$, de préférence encore d'environ $\leq 20\%$, et de manière hautement préférée d'environ $\leq 16,5\%$.

9. Procédé de l'une des revendications 6 à 8, **caractérisé en ce que** le groupe « mauvais » est déterminé par une proportion relative de pRCC d'environ $\geq 5\%$, de préférence d'environ $\geq 7,5\%$, de préférence encore d'environ $\geq 10\%$, de préférence encore d'environ $\geq 12\%$, et de manière hautement préférée d'environ $> 16,5\%$.

10. Procédé de l'une des revendications précédentes, **caractérisé en ce qu'**à l'étape (a), l'analyse implique l'utilisation d'un séquençage d'ARN, d'une méthode basée sur la PCR, d'une méthode basée sur une puce à ADN, d'une méthode basée sur l'hybridation et/ou d'une méthode basée sur des anticorps.

11. Utilisation de

- au moins un, de préférence au moins deux des gènes signature listés dans le Tableau 1,
- au moins un, de préférence au moins deux des gènes signature listés dans le Tableau 2, et
- au moins un, de préférence au moins deux des gènes signature listés dans le Tableau 3,

pour déterminer dans un échantillon biologique d'un sujet les proportions relatives de carcinome papillaire à cellules rénales (pRCC), de carcinome rénal à cellules claires (ccRCC), et de carcinome chromophobe rénal (chRCC), de préférence encore pour classer le sujet dans un groupe à risque de RCC.

**Fig. 1**

Fig. 2

**Fig. 3**

Fig. 4

Fig. 5

**Fig. 6**

EP 3 722 444 B1

Fig. 7

**Fig. 8**

**Fig. 9**

EP 3 722 444 B1

**Fig. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015131095 A

**Non-patent literature cited in the description**

- **INAMURA.** Trans-location Renal Cell Carcinoma: An Update on Clinicopathological and Molecular Features. *Int. J. Mol. Sci.,* 2017, vol. 9 (9), 1-11
- **C.J. RICKETTS et al.** The Cancer Genome Atlas Comprehensive Molecular Characterization of Renal Cell Carcinoma. *Cell Reports,* 2018, vol. 23 (1), 313-326
- **BÜTTNER et al.** Survival Prediction of Clear Cell Renal Cell Carcinoma Based on Gene Expression Similarity to the Proximal Tubule of the Nephron. *Eur. Urol.,* 2015, vol. 68 (6), 1016-1020
- **CHEN et al.** Multilevel Genomics-Based Taxonomy of Renal Cell Carcinoma. *Cell Reports,* 2016, vol. 14 (10), 2476-2489
- **SCHAEFFELER et al.** Metabolic and Lipidomic Reprogramming in Renal Cell Carcinoma Subtypes Reflects Regions of Tumor Origin. *Eur. Urol. Focus,* 2018
- **RINI et al.** A 16-Gene Assay to Predict Recurrence After Surgery in Localised Renal Cell Carcinoma: Development and Validation Studies. *Lancet Oncol.,* 2015, vol. 16 (6), 676-685
- **WANG et al.** Identification and Validation of a 44-Gene Expression Signature for the Classification of Renal Cell Carcinomas. *J. Exp. Clin. Cancer Res.,* 2017, vol. 36 (176), 1-11
- **S. S. SHEN-ORR ; R. GAUJOUX.** Computational Deconvolution: Extracting Cell Type-Specific Information from Heterogeneous Samples. *Current Opinion in Immunology,* 2013, vol. 25, 571-578
- **F. AVLIA COBOS et al.** Computational Deconvolution of Transcriptomics Data from Mixed Cell Populations. *Bioinformatics,* 2018, vol. 34, 1969-1979
- **A. R. ABBAS et al.** Deconvolution of Blood Microarray Data Identifies Cellular Activation Patterns in Systemic Lupus Erythema-tosus. *PloS one,* 2009, vol. 4, e6098
- **R. GAUJOUX ; C. SEOIGHE.** CellMix: A Comprehensive Toolbox for Gene ExpressionDeconvolution. *Bioinformatics (Oxford, England),* 2013, 1-2

- **K.A. FURGE et al.** Detection of DNA Copy Number Changes and Oncogenic Signaling Abnormalities from Gene Expression Data Reveals MYC Activation in High-Grade Papillary Renal Cell Carcinoma. *Cancer Res.,* 2007, vol. 67 (7), 3171-3176
- **M.-H. TAN et al.** Genomic Expression and Single-Nucleotide Polymorphism Profiling Discriminates Chromophobe Renal Cell Carcinoma and Oncocytoma. *BMC Cancer,* 2010, vol. 10, 196
- **S. PEÑA-LLOPIS et al.** BAP1 Loss Defines a New Class of Renal Cell Carcinoma. *Nat. Genet.,* 2012, vol. 44 (7), 751-759
- **M.V. YUSENKO et al.** High-resolution DNA Copy Number and Gene Expression Analyses Distinguish Chromophobe Renal Cell Carcinomas and Renal Oncocytomas. *BMC Cancer,* 2009, vol. 9, 152
- **T.H. HO et al.** Differential Gene Expression Profiling of Matched Primary Renal Cell Carcinoma and Metastases Reveals Upregulation of Extracellular Matrix Genes. *Ann. Oncol. Off. J. Eur. Soc. Med. Oncol.,* 2017, vol. 28 (3), 604-10
- **J. LIU et al.** An Integrated TCGA Pan-Cancer Clinical Data Resource to Drive High-Quality Survival Outcome Analytics. *Cell,* 2018, vol. 173 (2), 400-416.e11
- **P. FISEL et al.** DNA Methylation of the SLC16A3 Promoter Regulates Expression of the Human Lactate Transporter MCT4 in Renal Cancer with Consequences for Clinical Outcome. *Clin. Cancer Res.,* 2013, vol. 19 (18), 5170-5181
- **S. WINTER et al.** Methylomes of Renal Cell Lines and Tumors or Metastases Differ Significantly with Impact on Pharmacogenes. *Sci Rep.,* 2018, vol. 6 (1
- **Y. ZHAO ; R. SIMON.** Gene Expression Deconvolution in Clinical Samples. *Genome Med.,* 2010, vol. 2 (12), 93
- **Y. ZHONG ; Z. LIU.** Gene expression deconvolution in linear space. *Nat. Methods,* 2011, vol. 9 (1), 8-9
- **A.M. NEWMAN et al.** Robust enumeration of cell subsets from tissue expression profiles. *Nat. Methods,* 2015, vol. 12 (5), 453-457

- **K. YOSHIHARA et al.** Inferring tumour purity and stromal and immune cell admixture from expression data. *Nat. Commun.,* 2013, vol. 4, 2612

- **F. BÜTTNER et al.** Survival Prediction of Clear Cell Renal Cell Carcinoma Based on Gene Expression Similarity to the Proximal Tubule of the Nephron. *Eur. Urol.,* 2015, vol. 68 (6), 1016-1020